# EUROPEAN PATENT SPECIFICATION

(11) **EP 3 432 871 B1**
(45) Date of publication and mention of the grant of the patent: **15.11.2023**
(21) Application number: 17770981.3
(22) Date of filing: 21.03.2017
(51) Int. Cl.: A61K 31/192, A61K 31/485, A61K 9/20, A61K 45/06, A61P 17/04

(54) **TREATMENT OF UREMIC PRURITUS**
BEHANDLUNG VON URÄMISCHEM PRURITUS
TRAITEMENT DU PRURIT URÉMIQUE

(30) Priority: 21.03.2016 US 201662311134 P; 25.10.2016 US 201662412584 P
(43) Date of publication of application: 30.01.2019
(73) Proprietor: Trevi Therapeutics, Inc., New Haven, Connecticut 06510 (US)
(72) Inventor: SCIASCIA, Thomas, Belmont, Massachusetts 02478 (US); GOOD, Jennifer, Sandy Hook, Connecticut 06482 (US); HAWI, Amale, Ridgefield, Connecticut 06877 (US)
(74) Representative: Cooley (UK) LLP
(86) International application number: PCT/US2017/023398
(87) International publication number: WO 2017/165409

(56) References cited:
- WO-A1-2015/192071
- US-A1- 2009 093 509
- US-A1- 2009 247 635
- US-B1- 8 637 538
- AMALE HAWI ET AL: "Pharmacokinetics of nalbuphine hydrochloride extended release tablets in hemodialysis patients with exploratory effect on pruritus", BMC NEPHROLOGY, BIOMED CENTRAL, LONDON, GB, vol. 16, no. 1, 8 April 2015 (2015-04-08), page 47, XP021217012, ISSN: 1471-2369, DOI: 10.1186/S12882-015-0043-3
- "Nalbuphine ER Tablets in Hemodialysis Patients With Severe Uremic Pruritus: Multicenter, Randomized, Double-Blind, Placebo-Controlled Trial ED - Collins Allan J; Chan Christopher T", AMERICAN JOURNAL OF KIDNEY DISEASES, vol. 67, no. 5, 15 April 2016 (2016-04-15) , XP029510317, ISSN: 0272-6386, DOI: 10.1053/J.AJKD.2016.03.192
- "Long-Term Effects of Nalbuphine ER Tablets in Hemodialysis Patients With Uremic Pruritus: A Multicenter Open-Label Trial ED - Collins Allan J; Chan Christopher T", AMERICAN JOURNAL OF KIDNEY DISEASES, vol. 67, no. 5, 15 April 2016 (2016-04-15) , XP029510316, ISSN: 0272-6386, DOI: 10.1053/J.AJKD.2016.03.191

## Description

### CROSS-REFERENCE TO RELATED APPLICATIONS

The present application claims the benefit of priority to U.S. Provisional Application No. 62/311,134, filed on March 21, 2016, and U.S. Provisional Application No. 62/412,584, filed on October 25, 2016.

### FIELD OF THE INVENTION

The present invention relates to nalbuphine compositions for use for treating uremic pruritus in patients.

### BACKGROUND

Pruritus, or itch, is a sensation that stimulates the desire or reflex to scratch, which can be either generalized or localized. The cause of pruritus is not fully understood. Proposed contributors to the pathogenesis of pruritus may include anemia or other manifestation of erythropoietin deficiency, histamine release from skin mast cells, skin dryness, secondary hyperparathyroidism, hyperphosphatemia with increased calcium phosphate deposition in the skin and alterations in the endogenous opioidergic system with overexpression of opioid µ-receptors.

Pruritus is a frequently identified sign and symptoms of uremia ("uremic pruritus" (UP)) and is thus a common symptom in patients receiving hemodialysis. In terms of treatment options for UP, there have been a variety of medical interventions discussed and an effective treatment is still needed.

Amale Hawi et al., BMC Nephrology, vol.16(1),2015, p.47 discloses nalbuphine HCI extended release (ER) tablets to reduce itch severity in patients with uremic pruritus, whereby the patients received a single 30 mg dose on day 1 and doses were subsequently escalated to twice daily (BID) 30 mg, 60 mg, 120 mg, 180 mg over 13 days or to 240 mg BID over 15 days.

US8637538 discloses nalbuphine ER tablets for use in the treatment of uremic pruritus. Nalbuphine is administered at an initial dose of 15 to 60 mg twice a day and then titrated to an effective dose.

### SUMMARY OF THE INVENTION

Note that the references to methods of treatment in this description are to be interpreted as references to the compounds, pharmaceutical compositions and medicaments of the present invention for use in a method for treatment of the human (or animal) body by therapy.

The present invention, among other things, provides methods of treating pruritus comprising administering an effective amount of an anti-pruritus agent to a patient in need of such treatment. According to the invention, the anti-pruritus agent is nalbuphine or a pharmaceutically acceptable salt, solvate or ester thereof.

In some embodiments, the patient in need of a treatment of pruritus is a patient with uremic pruritus. In certain embodiments, the patient has moderate or severe uremic pruritus. In one embodiment, the patient in need of a treatment is a patient with compromised renal function. In yet another embodiment, the patient in need of a treatment is a patient with chronic kidney disease. In yet another embodiment, the patient in need of a treatment is a hemodialysis patient.

According to some embodiments of the present invention, the method of treating uremic pruritus comprises administering for at least a week to a patient in need thereof a daily dose of 240 mg of nalbuphine or a pharmaceutically acceptable salt, solvate or ester thereof. In some embodiments, 120 mg of the anti-pruritus agent is administered twice a day. In some embodiments, 240 mg of the anti-pruritus agent is administered once a day.

In some embodiments, the anti-pruritus agent is administered for 8 weeks. In some embodiments, the anti-pruritus agent is administered for 12 weeks. In some embodiments, the anti-pruritus agent is administered for 24 weeks.

In some embodiments, after the treatment the patient experiences a substantial reduction in itch compared to prior to the treatment.

According to the invention, the method of treating pruritus further includes a step of titrating the dose of the anti-pruritus agent for at least 2 weeks prior to the administration.

In certain embodiments, ascending doses of the anti-pruritus agent are administered during the titration until a steady state is achieved in the patient. In certain embodiments, ascending doses of the anti-pruritus agent are administered during the titration until an effective amount of 120 mg is achieved in the patient.

In one embodiment, the titration is initiated with a dose of about 15 mg once or twice a day. In another embodiment, the titration is initiated with a dose of about 30 mg once or twice a day. In certain embodiments, the titration comprises administering the anti-pruritus agent in increments ranging from about 15 mg to about 30 mg. In certain embodiments, titration twice a day is with an AM dosage and a PM dosage, wherein the PM dosage is higher than or the same as the AM dosage.

In accordance with some embodiments of the present invention, the rate of adverse events after the treatment with the anti-pruritus agent is substantially the same as the rate of adverse events after administering a placebo for the same period of time.

According to some embodiments of the present invention, clinical studies show that subjects treated with an anti-pruritus agent experience a statistically significant reduction of itch compared to subjects treated with a placebo. In some embodiments, the statistically significant reduction of itch is indicated by a p value of less than or equal to about 0.05. In some embodiments, the patient with moderate or severe baseline itch prior to the treatment experiences mild itch after the treatment.

According to some embodiments of the present invention, after the treatment the patient experiences a reduction of itch that is characterized by at least about a 30%, 40%, or 50% decline in worst itching intensity Numerical Rating Scale (NRS) value. In some embodiments, after the treatment the patient experiences a reduction of itch that is characterized by at least about a 30%, 40%, or 50% decline in average itching intensity Numerical Rating Scale (NRS) value.

According to some embodiments of the present invention, after the treatment the patient experiences a reduction of itch that is characterized by at least about a 10%, 20%, or 30% improvement in total Skindex-10 score. In some embodiments, after the treatment the patient experiences a reduction of itch that is characterized by at least about a 10%, 20%, or 30% improvement in Skindex-10 disease domain score. In some embodiments, after the treatment the patient experiences a reduction of itch that is characterized by at least about a 10%, 20%, or 30% improvement in Skindex-10 mood/emotional distress domain score. In some embodiments, after the treatment the patient experiences a reduction of itch that is characterized by at least about a 10%, 20%, or 30% improvement in Skindex-10 social functioning domain score.

According to some embodiments of the present invention, after the treatment the patient experiences a reduction of itch that is characterized by at least about a 20%, 30%, or 40% improvement in Itch Medical Outcomes Study (MOS) sleep scale.

In accordance with some embodiments of the present invention, the method of treating pruritus does not produce a substantial aquaretic effect. In accordance with some embodiments of the present invention, after the treatment the rate of infection of the patient is lower than that of the patient prior to the treatment. In accordance with some embodiments of the present invention, after the treatment the rate of muscoloskeletal complaints of the patient is lower than that of the patient prior to the treatment.

In some embodiments, the method of treating pruritus further includes administering at least one additional antipruritic drug. In certain embodiments, the at least one additional antipruritic drug is selected from the group consisting of antihistamines and corticosteroids.

In some embodiments, the anti-pruritus agent is in the form of an extended release oral dosage form.

In some embodiments, the anti-pruritus agent is administered in a formulation comprising nalbuphine hydrochloride, mannitol, hydroxypropyl cellulose, locust bean gum, xanthan gum, calcium sulfate dihydrate, and magnesium stearate.

The present methods, and advantages thereof, are further illustrated by the following non-limiting detailed description and Examples.

### BRIEF DESCRIPTION OF THE DRAWINGS

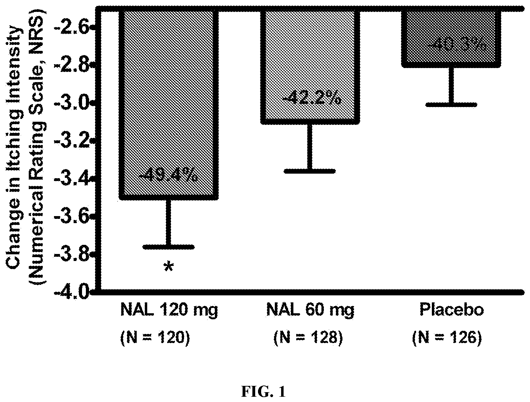

**FIG. 1** is a graphical representation of Primary Efficacy Endpoint - Change from Baseline to the Evaluation Period (last 2 treatment weeks) in the Worst Itching Numerical Rating Scale (range, 0 - 10; anchors at 0 "no itching"; 4-6 "moderate itching"; and 10 "worst possible itching"). NAL 120 = nalbuphine ER tablets 120 mg BID; NAL 60 = nalbuphine ER tablets 60 mg BID; *p=0.017 vs. placebo. Data presented are LS means and SEM.

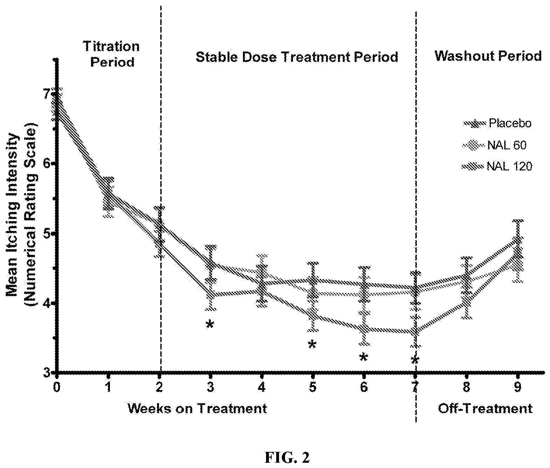

**FIG. 2** is a graphical representation of change in Worst Itching Intensity Numerical Rating Scale by Study Week. The range of the Numerical Rating Scale was 0 - 10 with anchors at 0 "no itching"; 4-6 "moderate itching"; and 10 "worst possible itching"). Data depicted are means (SEM). NAL 120 = nalbuphine ER tablets 120 mg BID; NAL 60 = nalbuphine ER tablets 60 mg BID; *p<0.05 vs. placebo.

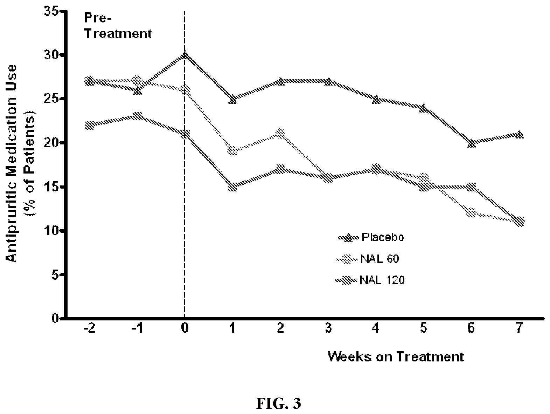

**FIG. 3** is a graphical representation of Percentage of Patients Taking Antipruritic Medications Over Time. NAL 120 = nalbuphine ER tablets 120 mg BID; NAL 60 = nalbuphine ER tablets 60 mg BID.

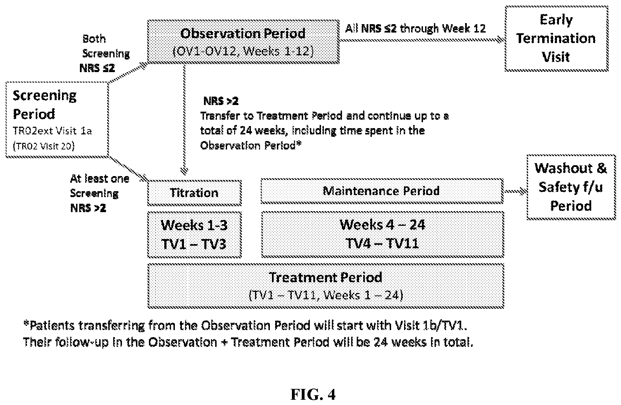

**FIG. 4** is an overall schematic of a Phase 2/3 extension study described in Example 3.

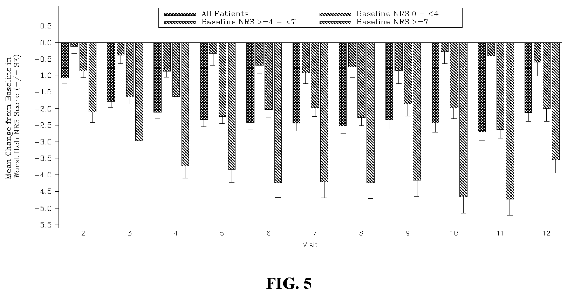

**FIG. 5** is a graphical representation of the mean change from baseline in worst itch NRS score by week for (1) all patients; (2) patients with a baseline itch NRS from zero to less than 4; (3) patients with a baseline itch NRS from greater than or equal to 4 but less than 7; and (4) patients with a baseline itch NRS greater than or equal to 7 in the Phase 2/3 extension study described in Example 3.

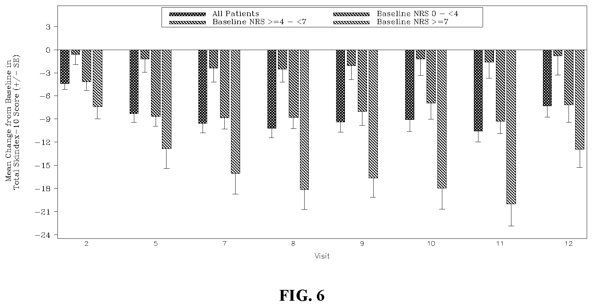

**FIG. 6** is a graphical representation of the mean change from baseline in total Skindex-10 score by week for (1) all patients; (2) patients with a baseline itch NRS from zero to less than 4; (3) patients with a baseline itch NRS from greater than or equal to 4 but less than 7; and (4) patients with a baseline itch NRS greater than or equal to 7 in the Phase 2/3 extension study described in Example 3.

### DEFINITIONS

The term "about" when immediately preceding a numerical value means a range of plus or minus 10% of that value, e.g., "about 50" means 45 to 55, "about 25,000" means 22,500 to 27,500, etc., unless the context of the disclosure indicates otherwise, or is inconsistent with such an interpretation. For example in a list of numerical values such as "about 49, about 50, about 55, ... ", "about 50" means a range extending to less than half the interval(s) between the preceding and subsequent values, e.g., more than 49.5 to less than 52.5. Furthermore, the phrases "less than about" a value or "greater than about" a value should be understood in view of the definition of the term "about" provided herein.

For convenience, certain terms employed in the specification, examples and claims are collected here. Unless defined otherwise, all technical and scientific terms used in this disclosure have the same meanings as commonly understood by one of ordinary skill in the art to which this disclosure belongs.

The terms "administer," "administering" or "administration" as used herein refer to either directly administering a compound or pharmaceutically acceptable salt or ester of the compound or a composition comprising the compound or pharmaceutically acceptable salt or ester of the compound to a patient.

The term "adverse event" (AE) as used herein is defined as any untoward medical occurrence in a clinical investigation patient reported on or after the first screening date. An AE does not necessarily have to have a causal relationship with the treatment. An AE can therefore be any unfavorable and unintended sign (including an abnormal laboratory finding), symptom whether or not related to the medicinal (investigational) product, or disease temporally associated with the use of a medicinal (investigational) product. Typical adverse events include nausea, vomiting, somnolence, dizziness and hallucination. In accordance with the present invention, the rate of adverse events after the treatment is substantially the same as the rate of adverse events after administering a placebo for the same period of time.

The term "carrier" as used herein encompasses carriers, excipients, and diluents, meaning a material, composition or vehicle, such as a liquid or solid filler, diluent, excipient, solvent or encapsulating material involved in carrying or transporting a pharmaceutical agent from one organ, or portion of the body, to another organ or portion of the body.

The term "disorder" is used in this disclosure to mean, and is used interchangeably with, the terms disease, condition, or illness, unless otherwise indicated.

The terms "effective amount" and "therapeutically effective amount" are used interchangeably in this disclosure and refer to an amount of a compound, or a salt, solvate or ester thereof, that, when administered to a patient, is capable of performing the intended result. For example, an effective amount of an anti-pruritic agent is that amount which is required to reduce at least one symptom of pruritus in a patient, e.g. the amount required to reduce the itching sensation in a patient. The actual amount that comprises the "effective amount" or "therapeutically effective amount" will vary depending on a number of conditions including, but not limited to, the severity of the disorder, the size and health of the patient, and the route of administration. A skilled medical practitioner can readily determine the appropriate amount using methods known in the medical arts.

The phrase "pharmaceutically acceptable" as used herein refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "salts" as used herein embraces pharmaceutically acceptable salts commonly used to form alkali metal salts of free acids and to form addition salts of free bases. The nature of the salt is not critical, provided that it is pharmaceutically acceptable. The term "salts" also includes solvates of addition salts, such as hydrates, as well as polymorphs of addition salts. Suitable pharmaceutically acceptable acid addition salts can be prepared from an inorganic acid or from an organic acid. Examples of such inorganic acids are hydrochloric, hydrobromic, hydroiodic, nitric, carbonic, sulfuric, and phosphoric acid. Appropriate organic acids can be selected from aliphatic, cycloaliphatic, aromatic, arylaliphatic, and heterocyclyl containing carboxylic acids and sulfonic acids, for example formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, p-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, cyclohexylaminosulfonic, algenic, 3-hydroxybutyric, galactaric and galacturonic acid.

The term "treating" as used herein with regard to a patient, refers to improving at least one symptom of the patient's disorder. Treating can be curing, improving, or at least partially ameliorating a disorder.

The term "therapeutic effect" as used herein refers to a desired or beneficial effect provided by the method and/or the composition. For example, the method for treating pruritus provides a therapeutic effect when the method reduces at least one symptom of pruritus, e.g., itching sensation, in a patient.

### DETAILED DESCRIPTION

According to the present invention, pruritus includes any itchy or pruritic condition, e.g., a sensation that causes the desire or reflex to scratch. Pruritus is a distressing hallmark of the uremic condition. Despite modem day dialytic management, 60% of dialysis patients experience itching and approximately 30-45% experience moderate or severe/extreme pruritus. Data from the Dialysis Practice Patterns Study derived from 12 countries and over 20,000 hemodialysis patients show that those with moderate-to-severe pruritus were more likely to feel washed out, to have poor sleep quality, physician-diagnosed depression, and a reduced quality of life than patients with no or mild pruritus. Among a United States cohort of over 70,000 dialysis patients, the severity of pruritus was associated with greater use of IV antibiotics, higher ESA and IV iron doses. All-cause mortality and infection-related mortality has also been reported to be higher in patients with moderate or severe pruritus compared with those with mild pruritus.

The sensation of itch and the activity of scratching are two different phenomena, even though they may be viewed as closely associated. Itch is a conscious sensation, which in some cases can be alleviated or ameliorated by scratching. However, patients suffering from uremic pruritus often scratch (perhaps out of reflex) in their sleep, when they cannot consciously experience the sensation of itch. This scratching during the sleep period in patients suffering from uremic pruritus can cause sleep interruption or sleep deprivation so severe that it is associated with a significant increase in mortality. The methods of the present invention can substantially reduce sleep deprivation (or alternatively stated, improve sleep) for uremic pruritus patients, for example as described in the studies disclosed herein. Furthermore, the methods of the present invention are effective in reducing both the perception of itch and scratching behavior in uremic pruritus patients.

In one aspect, the present invention provides a method of treating pruritus comprising administering an effective amount of an anti-pruritus agent for at least a week to a patient in need of such treatment, wherein the anti-pruritus agent is nalbuphine or a pharmaceutically acceptable salt, solvate or ester thereof. In accordance with some embodiments of the present invention, 120 mg of the anti-pruritus agent is administered twice a day. In some embodiments, 240 mg of the anti-pruritus agent is administered once a day.

In some embodiments, methods of the present invention are used for the treatment of uremic pruritus. In some embodiments, methods of the present invention are used for the treatment of a patient suffering from a pruritic condition selected from the group consisting of renal failure, hemodialysis, and/or peritoneal dialysis. In one embodiment, the patient in need of a treatment of uremic pruritus is a patient with compromised renal function. In yet another embodiment, the patient in need of a treatment of uremic pruritus is a patient with chronic kidney disease. In yet another embodiment, the patient in need of a treatment of uremic pruritus is a hemodialysis patient. In certain embodiments, Nalbuphine HCl is used or indicated for the treatment of itch in adult patients with end-stage-renal disease on hemodialysis with moderate to severe uremic pruritus.

In accordance with some embodiments of the present invention, the method provides a therapeutic effect without producing a substantial adverse event. In some embodiments, the rate of adverse events after the treatment with the anti-pruritus agent is substantially the same as the rate of adverse events after administering a placebo for the same period of time.

In accordance with some embodiments of the present invention, the method of treating pruritus does not produce a substantial aquaretic effect.

In accordance with some embodiments of the present invention, after the treatment the rate of infection of the patient, presumably caused by bacteria entering the body from scratching of the skin, is lower than that of the patient prior to the treatment.

### Nalbuphine

Nalbuphine as employed in the present methods can form a part of a pharmaceutical composition by combining nalbuphine, or a pharmaceutically acceptable salt, solvate or ester thereof, with a pharmaceutically acceptable carrier. Additionally, the compositions can include an additive selected from the group consisting of adjuvants, excipients, diluents, release-modifying agents and stabilizers. The composition can be an immediate release formulation, a delayed release formulation, a sustained release formulation or an extended release formulation.

Nalbuphine HCl (17-(cyclobutylmethyl)-4,5α-epoxymorphinian-3, 6α, 14-triol, hydrochloride) is a synthetic opioid. Structurally, nalbuphine is a derivative of 14-hydroxymorphine.

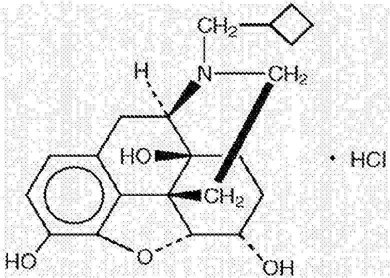

Nalbuphine HCl is currently available only as a generic medication in an injectable form. An injectable form of nalbuphine has been available as an approved drug formulation since 1978. Nubain^{®} was the innovator brand injectable form of nalbuphine on which the presently sold generic bioequivalent injectable formulations are based. The injectable formulation is currently approved for use in the relief of moderate to severe pain, a supplement to balanced anesthesia, for pre-operative and post-operative analgesia and obstetrical analgesia during labor and delivery.

The present invention also includes pharmaceutically acceptable esters of the anti-pruritus agent. The term "ester" denotes a derivative of the agent containing an ester functional group (as described herein), which is capable of releasing the agent when the ester form is administered to a patient. Release of the active ingredient occurs in vivo. Pharmaceutically acceptable esters can be prepared by techniques known to one skilled in the art. These techniques generally modify appropriate functional groups in a given compound. These modified functional groups however regenerate original functional groups by metabolism of the compound in vivo. Esters include compounds wherein a hydroxy, carboxylic, or a similar group is modified.

Suitable pharmaceutically acceptable esters for a hydroxyl group include inorganic esters such as phosphate esters and α-acyloxyalkyl ethers and related compounds which, as a result of in vivo hydrolysis of the ester, provide the parent hydroxy group. In vivo hydrolyzable ester forming groups for hydroxy include alkanoyl (e.g., C₁₋₁₀ linear, branched or cyclic alkyl), benzoyl, phenylacetyl and substituted benzoyl and phenylacetyl, alkoxycarbonyl (to give alkyl carbonate esters), dialkylcarbamoyl and N-(N,N-dialkylaminoethyl)-N-alkylcarbamoyl (to give carbamates), N,N-dialkylaminoacetyl and carboxy acetyl.

### Formulations

The methods of the present invention can employ various formulations for administration to patients, e.g., humans and animals in unit dosage forms, such as tablets, capsules, pills, powders, granules, sterile parenteral solutions or suspensions, and oral solutions or suspensions, and oil-water emulsions containing suitable quantities of an anti-pruritic agent, e.g., nalbuphine, or pharmaceutically acceptable salts or esters thereof.

Oral pharmaceutical dosage forms can be either solid or liquid. The solid dosage forms can be tablets, capsules, granules, and bulk powders. Types of oral tablets include compressed, chewable lozenges and tablets which can be enteric-coated, sugar-coated or film-coated. Capsules can be hard or soft gelatin capsules, while granules and powders can be provided in non-effervescent or effervescent form with the combination of other ingredients known to those skilled in the art. In other embodiments, the oral dosage form may be an osmotic-controlled release oral delivery system (OROS). In other embodiments, the oral dosage form may include matrix-embedded dosage forms or related devices. In some embodiments, the present oral dosage forms may include orally-disintegrating tablets.

Pharmaceutically acceptable carriers utilized in tablets include binders, lubricants, diluents, disintegrating agents, coloring agents, flavoring agents, and wetting agents.

Liquid oral dosage forms include aqueous solutions, emulsions, suspensions, solutions and/or suspensions reconstituted from non-effervescent granules and effervescent preparations reconstituted from effervescent granules.

Aqueous solutions include, for example, elixirs and syrups. Emulsions can be either oil-in water or water-in-oil. Elixirs are clear, sweetened, hydroalcoholic preparations. Pharmaceutically acceptable carriers used in elixirs include solvents. Syrups can be concentrated aqueous solutions of a sugar, for example, sucrose, and can contain a preservative. An emulsion is a two-phase system in which one liquid is dispersed in the form of small globules throughout another liquid. Pharmaceutically acceptable carriers used in emulsions are non-aqueous liquids, emulsifying agents and preservatives. Suspensions can use pharmaceutically acceptable suspending agents and preservatives. Pharmaceutically acceptable substances used in non-effervescent granules, to be reconstituted into a liquid oral dosage form, include diluents, sweeteners and wetting agents. Pharmaceutically acceptable substance used in effervescent granules, to be reconstituted into a liquid oral dosage form, can include organic acids and a source of carbon dioxide. Coloring and flavoring agents can be used in all of the above dosage forms.

Parenteral administration of the formulations of the present invention includes intravenous, subcutaneous and intramuscular administrations of immediate, sustained (e.g., depot), extended, and/or modified release formulations (e.g., as described herein). Preparations for parenteral administration include sterile solutions ready for injection, sterile dry soluble products ready to be combined with a solvent just prior to use, including hypodermic tablets, sterile suspensions ready for injection, sterile dry insoluble products ready to be combined with a vehicle just prior to use and sterile emulsions. The solutions can be either aqueous or nonaqueous. Pharmaceutically acceptable carriers used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

The concentration of the pharmaceutically active compound can be adjusted so that an injection provides an effective amount to produce the desired pharmacological effect. The exact dose depends on the age, weight and condition of the patient or animal, as is known in the art. The unit-dose parenteral preparations are packaged in an ampoule or a syringe with a needle. All preparations for parenteral administration must be sterile, as is known and practiced in the art. Illustratively, intravenous or intra-arterial infusion of a sterile aqueous solution containing an anti-pruritic agent is an effective mode of administration.

Pharmaceutical dosage forms for rectal administration can be rectal suppositories, capsules and tablets for systemic effect. Rectal suppositories as used herein mean solid bodies for insertion into the rectum which melt or soften at body temperature releasing the pharmacologically and/or therapeutically active ingredients contained in the composition of this invention. Pharmaceutically acceptable substances utilized in rectal suppositories are bases or vehicles and agents to raise the melting point. Examples of bases include cocoa butter (theobroma oil), glycerin-gelatin, carbowax, polyoxyethylene glycol and mixtures of mono-, di- and triglycerides of fatty acids. Combinations of the various bases can be used. Agents to raise the melting point of suppositories include spermaceti and wax. Rectal suppositories can be prepared either by the compressed method or by molding. The typical weight of a rectal suppository is about 2 to 3 gm. Tablets and capsules for rectal administration can be manufactured using the same pharmaceutically acceptable substance and by the same methods as for formulations for oral administration.

The compositions can be suspended in micronized or other suitable form or can be derivatized to produce a more soluble active product. The form of the resulting composition depends upon a number of factors, including the intended mode of administration and the solubility of the anti-pruritic agent in the selected carrier or vehicle. The effective concentration is sufficient for treating or alleviating pruritus, and can be empirically determined. The concentration is generally greater than the concentration for systemic administration of the compound.

The resulting mixture can be a solution, suspension, emulsion or the like, and can be formulated as a cream, gel, ointment, emulsion, solution, elixir, lotion, suspension, tincture, paste, foam, aerosol, irrigation, spray, suppository, bandage, or any other formulation suitable for topical or local administration. Modes of administration can include topical application to the skin, scalp, eyes, and/or nasal, buccal or sublingual mucosa.

Pharmaceutical and cosmetic carriers or vehicles suitable for administration of the compositions include any such carriers known to those skilled in the art to be suitable for the particular mode of administration. The anti-pruritic agent can be included in the carriers in amounts sufficient to exert a therapeutically useful effect without serious toxic effects on the treated individual.

To formulate these compositions, a weight fraction of an anti-pruritic agent is dissolved, suspended, dispersed or otherwise mixed in a selected vehicle at an effective concentration such that the pruritic condition is relieved or ameliorated. Generally, emollient or lubricating vehicles that help hydrate the skin are more preferred than volatile vehicles, such as ethanol, that dry the skin. Examples of suitable bases or vehicles for preparing compositions for use with human skin are petrolatum, petrolatum plus volatile silicones, lanolin, cold cream (USP), and hydrophilic ointment (USP).

The compositions employed in the present methods can relieve pruritus when applied to the skin. The composition can be administered topically to the affected area up to eight times per day, as needed, to provide reduction in and relief from itching. Relief can be temporary or permanent, and can even be evident after a single dose of the composition. When the composition is administered in a form other than a topical preparation, it should be administered in an amount sufficient to provide relief from pruritus that is within safety guidelines established by the FDA. Determining the appropriate amount to administer to a patient is within the skill of the person of ordinary skill in the art in association with teachings provided by the present invention.

Solutions of the compositions of this invention intended for topical administration contain an amount of the composition effective to deliver an anti-pruritic amount, typically at a concentration of between about 0.01% w/w to about 5% w/w. The balance of the solution is water, a suitable organic solvent or other suitable solvent or buffer. These compositions that are formulated as solutions or suspensions can be applied to the skin, or can be formulated as an aerosol or foam and applied to the skin as a spray-on. The aerosol compositions typically contain from 25% to 80% w/w, preferably from 30% to 50% w/w, of a suitable propellant. Gel compositions can be formulated by simply admixing a suitable thickening agent to the solution or suspension.

Solutions and suspensions can also be topically applied to the eyes and mucosa. Solutions, particularly those intended for ophthalmic use, can be formulated as 0.01%-10% w/w isotonic solutions, pH about 5-7, with appropriate salts, and preferably containing one or more of the compositions herein at a concentration of about 0.1% w/w, up to about 5% w/w or more. Suitable ophthalmic solutions are known in the art.

Compositions of solid forms intended for topical application can be formulated as stick-type compositions intended for application to the lips or other parts of the body. Such compositions contain an effective amount of an anti-pruritic agent, e.g. nalbuphine or a pharmaceutically acceptable salt, solvate or ester thereof. The amount of the anti-pruritic agent present is typically from about 0.01% w/w to about 5% w/w. The solids also contain from about 40% to 98% w/w, preferably from about 50% to 90% w/w, of emollients. This composition can further contain from 1% to 20% w/w, preferably from 5% to 15% w/w, of a suitable thickening agent, and, if desired or needed, emulsifiers and water or buffers.

In addition, the compositions, and preparations containing the compositions, can also be coated on bandages, mixed with bioadhesives, or included in dressings. Thus, combinations of bandages, bioadhesives, dressings and other such materials and the compositions formulated as described herein are provided.

### Sustained Release

Nalbuphine formulations that can be employed in the present methods include oral sustained release nalbuphine formulations as described in U.S. Provisional Pat. Appl. Nos. 60/772,466, 60/710,772, and 62/011,936; U.S. Pat. Appl. Nos. 11/509,347 (published as US 2007/0048376), 12/154,496 (published as US 2009/0030026), and 14/738,550; and PCT Appl. No. PCT/US2015/035650;

"Sustained release" or "extended release" means that the nalbuphine or pharmaceutically acceptable salt, solvate or ester thereof is released from the formulation at a controlled rate so that therapeutically beneficial blood levels (but below toxic levels) of the nalbuphine or pharmaceutically acceptable salt, solvate or ester thereof are maintained over an extended period of time. Alternatively, "sustained release" or "extended release" means that the desired pharmacologic effect is maintained over an extended period of time.

The half-life of nalbuphine injectable formulations (i.e., IV or IM or SC) has been reported to be relatively short, only about 2-3 hours. In some embodiments, the present methods can employ oral sustained release formulations of nalbuphine including an anti-pruritic effective amount of nalbuphine or a pharmaceutically acceptable salt, solvate or ester thereof. The oral sustained release formulations can provide a controlled release and a lower Cₘₐₓ of the anti-pruritus agent over a longer period than observed for bolus injections or immediate release oral formulations (e.g., at least about 8-12 hours, and in patients on dialysis, up to 14 hours or 22 hours). Reducing the frequency of dosing provides the potential for enhanced patient convenience and compliance with the present methods. The lower dosing frequency also has the potential to provide reduced side effects because the patient may be exposed to lower peak concentrations of agent over time.

Without wishing to be bound by a particular theory, the longer than expected duration of anti-pruritic effect is attributed to the enterohepatic recirculation of nalbuphine. Nalbuphine forms a glucuronic acid or other type of conjugated metabolite in vivo through enzymatic reaction with an enzyme system such as UDP-glucuronyl transferase. It is also possible that enterohepatic recirculation also occurs when parent drug in the bile is released from the gallbladder into the intestine and reabsorbed. Once formed, the conjugated nalbuphine product is thought to be transported into the gastrointestinal tract via biliary secretion whereby the drug conjugate is cleaved liberating nalbuphine, which can be reabsorbed from the intestine. The sustained release formulation can improve the duration of anti-pruritic effect, by more slowly releasing nalbuphine into the in vivo system and allowing more drug to be conjugated and therefore available for recirculation and later reabsorption from the intestine.

The present methods can employ compositions including nalbuphine or a pharmaceutically acceptable salt, solvate or ester thereof and a sustained release delivery system. The sustained release delivery system includes (i) at least one hydrophilic compound, at least one cross-linking agent, and at least one pharmaceutical diluent; (ii) at least one hydrophilic compound, at least one cross-linking agent, at least one pharmaceutical diluent, and at least one cationic cross-linking agent different from the first cross-linking agent; or (iii) at least one hydrophilic compound, at least one cationic cross-linking compound, and at least one pharmaceutical diluent. Alternatively, in other embodiments, the present methods can employ compositions including nalbuphine or a pharmaceutically acceptable salt, solvate or ester thereof and a sustained release delivery system, which may employ a hydrophobic compound in a sustained release system.

The nalbuphine can be homogeneously dispersed in the sustained release delivery system. In some embodiments, the nalbuphine or pharmaceutically acceptable salt, solvate or ester thereof is present in the composition in an amount of about 1 mg to about 240 mg; about 1 mg to about 150 mg; about 1 mg to about 125 mg; or about 1 mg to about 100 mg. In some embodiments, the nalbuphine or pharmaceutically acceptable salt, solvate or ester thereof is present in the composition in an amount of about 5 mg to about 80 mg; about 10 mg to about 70 mg; about 15 mg to about 60 mg; about 40 mg to about 80 mg; about 50 mg to about 70 mg; or about 45 mg to about 60 mg. In one embodiment, the nalbuphine or pharmaceutically acceptable salt, solvate or ester thereof is present in the composition in an amount of about 15 mg, about 20 mg, about 25 mg, about 30 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 110 mg, about 120 mg, about 130 mg, about 140 mg, about 150 mg, about 160 mg, about 170 mg, about 180 mg, about 190 mg, or about 240 mg. In another embodiment, the nalbuphine or pharmaceutically acceptable salt thereof is present in the composition in an amount of about 15 mg, about 30 mg, about 45 mg, about 60 mg, about 90 mg, about 120 mg, or about 180 mg.

In yet another embodiment, the nalbuphine or pharmaceutically acceptable salt thereof, e.g., HCL is present in the composition in an amount of about 15 mg, about 30 mg, about 60 mg, about 90 mg, about 120 mg, or about 180 mg.

In some embodiments, the sustained release delivery system is present in the composition in an amount from about 10 mg to about 420 mg; from about 25 mg to about 225 mg; from about 21 mg to about 198 mg; or from about 80 mg to about 200 mg; from about 80 mg to about 220 mg; from about 90 mg to about 210 mg; from about 100 mg to about 200 mg; from about 110 mg to about 190 mg; from about 120 mg to about 180 mg; from about 130 mg to about 170 mg; from about 140 mg to about 160 mg; from about 30 mg to about 60 mg; from about 60 mg to about 180 mg; from about 30 mg to about 180 mg, from about 75 mg to about 150 mg, from about 80 mg to about 160 mg, from about 90 mg to about 150 mg, from about 100 mg to about 140 mg, from about 110 mg to about 130 mg, from about 100 mg to about 300 mg, from about 200 mg to about 300 mg or from about 200 mg to about 250 mg. In one embodiment, the sustained release delivery system is present in the composition in an amount from about 75 mg to about 150 mg.

In some embodiments, the sustained release delivery system is present in the composition in an amount of about 30 mg, about 60 mg, about 75 mg, about 80 mg, about 90 mg, about 100 mg, about 110 mg, about 112 mg, about 115 mg, about 117 mg, about 120 mg, about 125 mg, about 130 mg, about 135 mg, about 140 mg, about 145 mg, about 150 mg, about 160 mg, about 170 mg, about 180 mg, about 190 mg, about 200 mg, about 210 mg, about 220 mg, about 225 mg, about 230 mg, about 240 mg, about 250 mg, about 260 mg, about 270 mg, about 280 mg, about 300 mg, about 320 mg, about 340 mg, about 360 mg, about 380 mg, about 400 mg or about 420 mg. In another embodiment, the sustained release delivery system is present in the composition in an amount of about 112 mg.

The ratio of nalbuphine or pharmaceutically acceptable salt, solvate or ester thereof in the compositions to the sustained release delivery system is generally from about 4:1 to about 1:25. In some embodiments, the ratio of nalbuphine or pharmaceutically acceptable salt, solvate or ester thereof to the sustained release delivery system is generally from about 2.5:1 to about 1:4. In some embodiments, the ratio of nalbuphine or pharmaceutically acceptable salt, solvate or ester thereof to the sustained release delivery system is generally from about 5:1 to about 1:5, about 4:1 to about 1:4, about 3:1 to about 1:3, about 2:1 to about 1:2, about 1:1 to about 1:5, about 1:1 to about 1:4, about 1:1 to about 1:3, about 1:1 to about 1.2, and about 1:2 to about 1:3. In some embodiments, the ratio of nalbuphine or pharmaceutically acceptable salt, solvate or ester thereof to the sustained release delivery system is about 1:1, about 1:2, about 1:2.5, about 1:3, about 1:4, or about 1:5.

In one embodiment, at least one hydrophilic compound is present in the sustained release delivery system in an amount of about 5% to about 80% by weight; the at least one cross-linking agent is present in the sustained release delivery system in an amount of about 0.5% to about 80% by weight; and the at least one pharmaceutical diluent is present in the sustained release delivery system in an amount of about 20% to about 80% by weight. In another embodiment, the at least one hydrophilic compound is present in the sustained release delivery system in an amount of about 8% to about 31% by weight; the at least one cross-linking agent is present in the sustained release delivery system in an amount of about 12% to about 47% by weight; and the at least one pharmaceutical diluent is present in the sustained release delivery system in an amount of about 20% to about 78% by weight. In another embodiment, the at least one hydrophilic compound is present in the sustained release delivery system in an amount of about 10% to about 20% by weight; the at least one cross-linking agent is present in the sustained release delivery system in an amount of about 15% to about 25% by weight; and the at least one pharmaceutical diluent is present in the sustained release delivery system in an amount of about 50% to about 85% by weight. In some embodiments, the at least one hydrophilic compound is present in the sustained release delivery system in an amount of about 8%, about 9%, about 10%, about 11%, about 12%, about 13%, about 14%, about 15%, about 16%, about 17%, about 18%, about 19%, about 20%, about 22%, about 24%, about 26%, about 28%, about 30%, about 32%, about 34%, or about 36% by weight; the at least one cross-linking agent is present in the sustained release delivery system in an amount of about 10%, about 11%, about 12%, about 13%, about 14%, about 15%, about 16%, about 17%, about 18%, about 19%, about 20%, about 22%, about 24%, about 26%, about 28%, about 30%, about 32%, about 33%, about 34%, or about 35% by weight; and the at least one pharmaceutical diluent is present in the sustained release delivery system in an amount of about 40%, about 45%, about 50%, about 55%, about 60%, about 65%, about 70%, about 80%, or about 85% by weight.

In some embodiments, the at least one hydrophilic compound is present in the sustained release delivery system in an amount of about 10%, about 11%, about 12%, about 13%, about 14%, about 15%, about 16%, about 17%, about 18%, about 19%, or about 20% by weight; the at least one cross-linking agent is present in the sustained release delivery system in an amount of about 15%, about 16%, about 17%, about 18%, about 19%, about 20%, or about 22% by weight; and the at least one pharmaceutical diluent is present in the sustained release delivery system in an amount of about 55%, about 60%, about 65%, about 70%, about 80%, or about 85% by weight. In one embodiment, the at least one hydrophilic compound is present in the sustained release delivery system in an amount of about 8%, about 12%, or about 20% by weight; the at least one cross-linking agent is present in the sustained release delivery system in an amount of about 12%, about 18%, or about 30% by weight; and the at least one pharmaceutical diluent is present in the sustained release delivery system in an amount of about 40%, about 60%, or about 70% by weight.

In one embodiment, nalbuphine is in the form of any pharmaceutically acceptable salt known in the art. Exemplary pharmaceutically acceptable salts include without limitation hydrochloric, sulfuric, nitric, phosphoric, hydrobromic, maleic, malic, ascorbic, citric, tartaric, pamoic, lauric, stearic, palmitic, oleic, myristic, lauryl sulfuric, napthalenesulfonic, linoleic, linolenic acid, and the like. One embodiment includes the hydrochloride salt of nalbuphine.

The sustained release delivery system includes at least one hydrophilic compound. The hydrophilic compound preferably forms a gel matrix that releases the nalbuphine or the pharmaceutically acceptable salt, solvate or ester thereof at a sustained rate upon exposure to liquids. The rate of release of the nalbuphine or the pharmaceutically acceptable salt, solvate or ester thereof from the gel matrix depends on the drug's partition coefficient between the components of the gel matrix and the aqueous phase within the gastrointestinal tract. The weight ratio of nalbuphine to hydrophilic compound is generally in the range of about 10:1 to about 1:10, about 9:1 to about 1:9, about 8:1 to about 1:8, about 7:1 to about 1:7, about 6:1 to about 1:6, about 5:1 to about 1:5, about 4:1 to about 1:4, about 3:1 to about 1:3, and about 2:1 to about 1:2. In some embodiments, the weight ratio of nalbuphine to hydrophilic compound is in the range of about 10:1 to about 1:1, about 10:1 to about 2:1, about 9:1 to about 1:1, about 8:1 to about 1:1, about 7:1 to about 1:1, about 6:1 to about 1:1, about 5:1 to about 1:1, about 4:1 to about 1:1, about 3:1 to about 1:1, and about 2:1 to about 1:1. In some embodiments, the weight ratio of nalbuphine to hydrophilic compound is in the range of about 6:1 to about 1:1, about 5:1 to about 2:1, about 4:1 to about 3:1, about 4:1 to about 2:1, and about 5:1 to about 2:1. In some embodiments, the weight ratio of nalbuphine to hydrophilic compound is about 1:5, about 1:4.5, about 1:4.4, about 1:4, about 1:3.5, about 1:3.3, about 1:3, about 1:2.5, about 1:2, about 1:1, and about 1:1.5.

The sustained release delivery system generally includes the hydrophilic compound in an amount of about 5% to about 80% by weight. In some embodiments, the sustained release delivery system generally includes the hydrophilic compound in an amount of about 5% to about 30%, about 8% to about 31%, about 10% to about 20%, about 20% to about 60%, or about 40% to about 60% by weight. In one embodiment, the sustained release delivery system includes the hydrophilic compound in an amount of about 8% to about 31% by weight. In one embodiment, the sustained release delivery system includes the hydrophilic compound in an amount of about 10% to about 20% by weight. In some embodiments, the sustained release delivery system includes the hydrophilic compound in an amount of about 10%, about 11%, about 12%, about 13%, about 14%, about 15%, about 16%, about 17%, about 18%, about 19%, or about 20% by weight. In one embodiment, the sustained release delivery system includes the hydrophilic compound in an amount of about 12% by weight. In one embodiment, the sustained release delivery system includes the hydrophilic compound in an amount of about 8% by weight. In one embodiment, the sustained release delivery system includes the hydrophilic compound in an amount of about 20% by weight. In one embodiment, the sustained release delivery system includes the hydrophilic compound in an amount of about 28% by weight.

The hydrophilic compound is any compound known in the art to be hydrophilic. Exemplary hydrophilic compounds include without limitation gums, cellulose ethers, polyvinyl pyrrolidone, protein-derived compounds, and mixtures thereof. Exemplary gums include without limitation heteropolysaccharide gums and homopolysaccharide gums, such as xanthan, tragacanth, pectins, acacia, karaya, alginates, agar, guar, hydroxypropyl guar, carrageenan, locust bean gums, and gellan gums. Exemplary cellulose ethers include without limitation hydroxyalkyl celluloses and carboxyalkyl celluloses. In some embodiments, cellulose ethers include hydroxyethyl celluloses, hydroxypropyl celluloses, hydroxypropylmethyl-celluloses, carboxy methylcelluloses, and mixtures thereof. In some embodiments, the hydrophilic compound is a gum. In other embodiments, the hydrophilic compound is a heteropolysaccharide gum. In further embodiments, the hydrophilic compound is a xanthan gum or derivative thereof. Derivatives of xanthan gum include without limitation, for example, deacylated xanthan gum, the carboxymethyl esters of xanthan gum, and the propylene glycol esters of xanthan gum.

In another aspect, the sustained release delivery system further includes at least one cross-linking agent. In one embodiment, the cross-linking agent is a compound that is capable of cross-linking the hydrophilic compound to form a gel matrix in the presence of liquids. As used herein, "liquids" includes, for example, gastrointestinal fluids and aqueous solutions, such as those used for in vitro dissolution testing. The sustained release delivery system generally includes the cross-linking agent in an amount of about 0.5% to about 80% by weight. In one embodiment, the sustained release delivery system generally includes the cross-linking agent in an amount of about 12% to about 47% by weight. In another embodiment, the sustained release delivery system generally includes the cross-linking agent in an amount of about 20% to about 30% by weight. In one embodiment, the sustained release delivery system generally includes the cross-linking agent in an amount of about 15% to about 25% by weight. In some embodiments, the at least one cross-linking agent is present in the sustained release delivery system in an amount of about 15%, about 16%, about 17%, about 18%, about 19%, about 20%, about 21%, about 22%, about 23%, about 24%, or about 25% by weight. In one embodiment, the sustained release delivery system includes the cross-linking agent in an amount of about 18% by weight. In one embodiment, the sustained release delivery system includes the cross-linking agent in an amount of about 12% by weight. In one embodiment, the sustained release delivery system includes the cross-linking agent in an amount of about 30% by weight. In one embodiment, the sustained release delivery system includes the cross-linking agent in an amount of about 42% by weight.

Exemplary cross-linking agents include homopolysaccharides. Exemplary homopolysaccharides include without limitation galactomannan gums, such as guar gum, hydroxypropyl guar gum, and locust bean gum. In some embodiments, the cross-linking agent is a locust bean gum or a guar gum. In other embodiments, the cross-linking agent is an alginic acid derivative or hydrocolloid.

In some embodiments, when the sustained release delivery system includes at least one hydrophilic compound and at least one cross-linking agent, the weight ratio of hydrophilic compound to cross-linking agent is from about 1:9 to about 9:1, about 1:8 to about 8:1, about 1:7 to about 7:1, about 1:6 to about 6:1, about 1:5 to about 5:1, about 1:4 to about 4:1, about 1:3 to about 3:1, or about 1:2 to about 2:1. In some embodiments, the weight ratio of hydrophilic compound to cross-linking agent is about 1:5, about 1:4.5, about 1:4, about 1:3.5, about 1:3, about 1:2.5, about 1:2, about 1:1.5, and about 1:1.

When the sustained release delivery system includes at least one hydrophilic compound and at least one cross-linking agent, the weight ratio of the nalbuphine or pharmaceutically acceptable salt, solvate or ester thereof to the sum of the at least one hydrophilic compound and the at least one cross-linking agent is from about 10:1 to about 1:10, from about 9:1 to about 1:9, from about 8:1 to about 1:8, from about 7:1 to about 1:7, from about 6:1 to about 1:6, from about 5:1 to about 1:5, from about 4:1 to about 1:4, from about 3:1 to about 1:3, or from about 2:1 to about 1:2. In some embodiments, the weight ratio of the nalbuphine or pharmaceutically acceptable salt, solvate or ester thereof to the sum of the at least one hydrophilic compound and the at least one cross-linking agent is from about 4:1 to about 1:1, from about 4:1 to about 1:1.5, from about 3:1 to about 1:1, or from about 2:1 to about 1:1. In one embodiment, the ratio of the nalbuphine or pharmaceutically acceptable salt, solvate or ester thereof to the sum of the at least one hydrophilic compound and the at least one cross-linking agent is about 5:1, about 4:1 (i.e., 1:0.25), about 3.5:1, about 3:1, about 2.5:1, about 2:1 (i.e., 1:0.5), about 1.9:1, about 1.8:1, about 1.7:1, about 1.6:1, about 1.5:1, about 1.4:1, about 1.3:1, about 1.2:1, about 1.1:1, about 1:1, about 1:1.5, about 1:2, about 1:3, about 1:4, and about 1:5.

The sustained release delivery system further includes one or more pharmaceutical diluents known in the art. Exemplary pharmaceutical diluents include without limitation monosaccharides, disaccharides, polyhydric alcohols and mixtures thereof. In some embodiments, pharmaceutical diluents include, for example, starch, mannitol, lactose, dextrose, sucrose, microcrystalline cellulose, sorbitol, xylitol, fructose, and mixtures thereof. In some embodiments, the pharmaceutical diluent is water-soluble. Nonlimiting examples of water-soluble pharmaceutical diluents include lactose, dextrose, sucrose, or mixtures thereof. The weight ratio of pharmaceutical diluent to hydrophilic compound is generally from about 1:9 to about 9:1, from about 1:8 to about 8:1, from about 1:7 to about 7:1, from about 1:6 to about 6:1, from about 1:5 to about 5:1, from about 1:4 to about 4:1, from about 1:3 to about 3:1, or from about 1:2 to about 2:1. In some embodiments, the weight ratio of pharmaceutical diluent to hydrophilic compound is generally from about 9:1 to about 1:1.5. In some embodiments, the weight ratio of pharmaceutical diluent to hydrophilic compound is about 9:1, about 8.75:1, about 8.5:1, about 8.25:1, about 8:1, about 7.5:1, about 7:1, about 6.5:1, about 6:1, about 5.5:1, about 5:1, about 4.5:1, about 4:1, about 3.5:1, about 3:1, about 2.5:1, about 2:1, about 1.5:1, or about 1:1.

The sustained release delivery system generally includes one or more pharmaceutical diluents in an amount of about 20% to about 80%, about 30% to about 70%, about 40% to about 70%, or about 40% to about 60%. In one embodiment, the sustained release delivery system includes one or more pharmaceutical diluents in an amount of about 20% to about 70% by weight. In one embodiment, the sustained release delivery system includes one or more pharmaceutical diluents in an amount of about 50% to about 85% by weight. In some embodiments, the sustained release delivery system includes one or more pharmaceutical diluents in an amount of about 55%, about 60%, about 65%, about 70%, about 80%, or about 85% by weight. In one embodiment, the sustained release delivery system includes one or more pharmaceutical diluents in an amount of about 20% by weight. In one embodiment, the sustained release delivery system includes one or more pharmaceutical diluents in an amount of about 30% by weight. In one embodiment, the sustained release delivery system includes one or more pharmaceutical diluents in an amount of about 40% by weight. In one embodiment, the sustained release delivery system includes one or more pharmaceutical diluents in an amount of about 50% by weight. In one embodiment, the sustained release delivery system includes one or more pharmaceutical diluents in an amount of about 60% by weight. In one embodiment, the sustained release delivery system includes one or more pharmaceutical diluents in an amount of about 70% by weight.

In a further aspect, the sustained release delivery system includes one or more cationic cross-linking compounds. In some embodiments, the one or more cationic cross-linking compounds are used instead of the cross-linking agent. In some embodiments, the one or more cationic cross-linking compounds are used in addition to the cross-linking agent. In one embodiment, the one or more cationic cross-linking compounds are used in an amount sufficient to cross-link the hydrophilic compound to form a gel matrix in the presence of liquids. In some embodiments, the one or more cationic cross-linking compounds are present in the sustained release delivery system in an amount of about 0.5% to about 30%, about 0.5% to about 25%, about 0.5% to about 20%, about 0.5% to about 15%, about 0.5% to about 10%, or about 0.5% to about 5% by weight. In some embodiments, the one or more cationic cross-linking compounds are present in the sustained release delivery system in an amount of about 5% to about 20%, about 5% to about 15%, about 6% to about 14%, about 7% to about 13%, about 8% to about 12%, or about 9% to about 11% by weight. In some embodiments, the one or more cationic cross-linking compounds are present in the sustained release delivery system in an amount of about 5%, about 6%, about 7%, about 8%, about 9%, about 10%, about 11%, about 12%, about 13%, about 14%, or about 15% by weight. In one embodiment, the cationic cross-linking compound is present in the sustained release delivery system in an amount of about 10% by weight.

Exemplary cationic cross-linking compounds include without limitation monovalent metal cations, multivalent metal cations, and inorganic salts, including alkali metal and/or alkaline earth metal sulfates, chlorides, borates, bromides, citrates, acetates, lactates, and mixtures thereof. For example, the cationic cross-linking compound include without limitation one or more of calcium sulfate, sodium chloride, potassium sulfate, sodium carbonate, lithium chloride, tripotassium phosphate, sodium borate, potassium bromide, potassium fluoride, sodium bicarbonate, calcium chloride, magnesium chloride, sodium citrate, sodium acetate, calcium lactate, magnesium sulfate, sodium fluoride, or mixtures thereof.

When the sustained release delivery system includes at least one hydrophilic compound and at least one cationic cross-linking compound, the weight ratio of hydrophilic compound to cationic cross-linking compound ranges from about 1:9 to about 9:1, from about 1:8 to about 8:1, from about 1:7 to about 7:1, from about 1:6 to about 6:1, from about 1:5 to about 5:1, from about 1:4 to about 4:1, from about 1:3 to about 3:1, or from about 1:2 to about 2:1. In one embodiment, the weight ratio of hydrophilic compound to cationic cross-linking compound ranges from about 1:3 to about 3:1. In some embodiments, the weight ratio of hydrophilic compound to cationic cross-linking compound is about 3:1, about 2.75:1, about 2.5:1, about 2.25:1, about 2:1, about 1.8:1, about 1.6:1, about 1.4:1, about 1.2:1, about 1:1, about 1:1.25, about 1:1.5, or about 1:2. In one embodiment, the weight ratio of hydrophilic compound to cationic cross-linking compound is about 1:1.25. In one embodiment, the weight ratio of hydrophilic compound to cationic cross-linking compound is about 1.2:1. In one embodiment, the weight ratio of hydrophilic compound to cationic cross-linking compound is about 2:1. In one embodiment, the weight ratio of hydrophilic compound to cationic cross-linking compound is about 2.8:1.

In one embodiment, the at least one hydrophilic compound is present in the sustained release delivery system in an amount of about 5% to about 80% by weight; the at least one cationic cross-linking agent is present in the sustained release delivery system in an amount of about 0.5% to about 30% by weight; and the at least one pharmaceutical diluent is present in the sustained release delivery system in an amount of about 20% to about 80% by weight. In another embodiment, the at least one hydrophilic compound is present in the sustained release delivery system in an amount of about 8% to about 30% by weight; the at least one cationic cross-linking agent is present in the sustained release delivery system in an amount of about 10% by weight; and the at least one pharmaceutical diluent is present in the sustained release delivery system in an amount of about 20% to about 70% by weight. In another embodiment, the at least one hydrophilic compound is present in the sustained release delivery system in an amount of about 5% to about 30% by weight; the at least one cationic cross-linking agent is present in the sustained release delivery system in an amount of about 5% to about 20% by weight; and the at least one pharmaceutical diluent is present in the sustained release delivery system in an amount of about 20% to about 85% by weight. In another embodiment, the at least one hydrophilic compound is present in the sustained release delivery system in an amount of about 10% to about 20% by weight; the at least one cationic cross-linking agent is present in the sustained release delivery system in an amount of about 5% to about 15% by weight; and the at least one pharmaceutical diluent is present in the sustained release delivery system in an amount of about 50% to about 85% by weight.

In some embodiments, the at least one hydrophilic compound is present in the sustained release delivery system in an amount of about 8%, about 9%, about 10%, about 11%, about 12%, about 13%, about 14%, about 15%, about 16%, about 17%, about 18%, about 19%, about 20%, about 22%, about 24%, about 26%, about 28%, or about 30% by weight; the at least one cationic cross-linking agent is present in the sustained release delivery system in an amount of about 5%, about 6%, about 7%, about 8%, about 9%, about 10%, about 11%, about 12%, about 13%, about 14%, about 15%, about 16%, about 17%, about 18%, about 19%, or about 20%, by weight; and the at least one pharmaceutical diluent is present in the sustained release delivery system in an amount of about 40%, about 45%, about 50%, about 55%, about 60%, about 65%, about 70%, about 80%, or about 85% by weight. In one embodiment, the at least one hydrophilic compound is present in the sustained release delivery system in an amount of about 10%, about 11%, about 12%, about 13%, about 14%, about 15%, about 16%, about 17%, about 18%, about 19%, or about 20% by weight; the at least one cationic cross-linking agent is present in the sustained release delivery system in an amount of about 5%, about 6%, about 7%, about 8%, about 9%, about 10%, about 11%, about 12%, about 13%, about 14%, about 15%, by weight; and the at least one pharmaceutical diluent is present in the sustained release delivery system in an amount of about 55%, about 60%, about 65%, about 70%, about 80%, or about 85% by weight. In one embodiment, the at least one hydrophilic compound is present in the sustained release delivery system in an amount of about 8%, about 12%, or about 20% by weight; the at least one cationic cross-linking agent is present in the sustained release delivery system in an amount of about 10%, about 12%, or about 14% by weight; and the at least one pharmaceutical diluent is present in the sustained release delivery system in an amount of about 40%, about 60%, or about 70% by weight.

In one embodiment, the sustained release delivery system includes about 0.5% to about 80% locust bean gum, about 5% to about 80% xanthan gum, about 20% to about 80% mannitol and about 0.5% to 80% calcium sulfate dihydrate. In one embodiment, the sustained release delivery system includes about 12% to about 47% locust bean gum, about 8% to about 31% xanthan gum, about 20% to about 78% mannitol and about 0.5% to 25% calcium sulfate dihydrate. In one embodiment, the sustained release delivery system includes about 15% to about 25% locust bean gum, about 10% to about 20% xanthan gum, about 50% to about 85% mannitol and about 5% to 15% calcium sulfate dihydrate. In one embodiment, the sustained release delivery system includes about 18% locust bean gum, about 12% xanthan gum, about 60% mannitol and about 10% calcium sulfate dihydrate. In one embodiment, the sustained release delivery system includes about 12% locust bean gum, about 8% xanthan gum, about 70% mannitol and about 10% calcium sulfate dihydrate. In one embodiment, the sustained release delivery system includes about 20% locust bean gum, about 30% xanthan gum, about 40% mannitol and about 10% calcium sulfate dihydrate. In one embodiment, the sustained release delivery system includes about 30% locust bean gum, about 20% xanthan gum, about 40% mannitol and about 10% calcium sulfate dihydrate. In one embodiment, the sustained release delivery system includes about 42% locust bean gum, about 28% xanthan gum, about 20% mannitol and about 10% calcium sulfate dihydrate.

Two properties of the components of this sustained release system (e.g., the at least one hydrophilic compound and the at least one cross-linking agent; or the at least one hydrophilic compound and at least one cationic cross-linking compound) are that it forms a gel matrix upon exposure to liquids are fast hydration of the compounds/agents and the ability to form a gel matrix having a high gel strength. These two properties, which are needed to achieve a slow release gel matrix, are maximized by the particular combination of compounds (e.g., the at least one hydrophilic compound and the at least one cross-linking agent; or the at least one hydrophilic compound and the at least one cationic cross-linking compound). For example, hydrophilic compounds (e.g., xanthan gum) have excellent water-wicking properties that provide fast hydration. The combination of hydrophilic compounds with materials that are capable of cross-linking the rigid helical ordered structure of the hydrophilic compound (e.g., cross-linking agents and/or cationic cross-linking compounds) thereby acts synergistically to provide a higher than expected viscosity (i.e., high gel strength) of the gel matrix.

In some embodiments, the sustained release compositions are further admixed with one or more wetting agents (e.g., polyethoxylated castor oil, polyethoxylated hydrogenated castor oil, polyethoxylated fatty acid from castor oil, polyethoxylated fatty acid from hydrogenated castor oil) one or more lubricants (e.g., magnesium stearate, sodium stearyl fumarate, and the like), one or more buffering agents, one or more colorants, and/or other conventional ingredients.

In some embodiments compositions employed in the present methods can contain additional pharmaceutical excipients. For example, in certain embodiments, fumaric acid can be added to the formulations described herein.

In other embodiments, a non-functional coating, e.g., Opadry^{®}, can be added to the compositions described herein.

In some embodiments, the compositions described herein further include a second hydrophilic compound. In some embodiments, the second hydrophilic compound is a cellulose ether. In some embodiments, the second hydrophilic compound is a hydroxyalkyl cellulose or a carboxyalkyl cellulose. In some embodiments, the second hydrophilic compound is a hydroxyethyl cellulose, a hydroxypropyl cellulose, a hydroxypropylmethylcellulose, a carboxy methylcellulose, or a mixture thereof. In some embodiments, the second hydrophilic is an ethyl cellulose or wax (e.g., including without limitation cetyl alcohol, stearyl alcohol, white wax, or carnauba wax). The second hydrophilic compound is present in the formulation in an amount ranging from about 5% to about 45%, about 5% to about 25%, about 10% to about 20%, or 12% to about 18% by weight. In some embodiments, the second hydrophilic compound is present in the formulation in an amount of about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 30%, about 35%, about 40%, or about 45%.

In some embodiments, the weight ratio of the second hydrophilic compound to the nalbuphine or pharmaceutically acceptable salt, solvate or ester ranges from about 5:1 to about 1:5, about 4:1 to about 1:4, about 3:1 to about 1:3, about 2:1 to about 1:2, about 1:1 to about 1:3, or about 1:1 to about 1:2. In some embodiments, the weight ratio of the second hydrophilic compound to the nalbuphine or pharmaceutically acceptable salt, solvate or ester is about 5:1, about 4:1, about 3:1, about 2:1, about 1:1, about 1:2, about 1:3, about 1:4, or about 1:5.

In some embodiments, the weight ratio of the second hydrophilic compound to the sustained release delivery system ranges from about 10:1 to about 1:10, about 8:1 to about 1:8, about 6:1 to about 1:6, about 4:1 to about 1:4, about 2:1 to about 1:3, about 1:1 to about 1:10, about 1:1 to about 1:6, or about 1:2 to about 1:6. In some embodiments, the weight ratio of the second hydrophilic compound to the sustained release delivery system is about 10:1, about 8:1, about 6:1, about 4:1, about 2:1, about 1:1, about 1:1.5, about 1:2, about 1:2.5, about 1:3, about 1:4, about 1:5, about 1:6, about 1:7, about 1:8, about 1:9 or about 1:10.

In some embodiments, the oral sustained release solid dosage formulations including from about 1 mg to 200 mg nalbuphine hydrochloride and about 10 mg to about 420 mg of a sustained release delivery system. In these embodiments, the sustained release delivery system includes about 12% to about 42% locust bean gum; about 8.0% to about 28% xanthan gum; about 20% to about 70% mannitol; and about 5% to about 20% calcium sulfate dihydrate. In some embodiments, the present methods can employ oral sustained release solid dosage formulations including from about 5 mg to about 80 mg nalbuphine hydrochloride and about 80 mg to about 360 mg of a sustained release delivery system. In some embodiments, the present methods can employ oral sustained release solid dosage formulations including from about 50 mg to about 150 mg nalbuphine hydrochloride and about 100 mg to about 300 mg of a sustained release delivery system.

In some embodiments, the present methods employ oral sustained release solid dosage formulations including about 15 mg nalbuphine hydrochloride, and from about 25 mg to about 225 mg, for example about 195 mg, of a sustained release delivery system. In these embodiments, the sustained release delivery system includes about 14% locust bean gum; about 9% xanthan gum; about 47% mannitol; and about 8% calcium sulfate dihydrate.

In some embodiments, the present methods employ oral sustained release solid dosage formulations including about 30 mg nalbuphine hydrochloride, and from about 25 mg to about 225 mg, for example about 180 mg, of a sustained release delivery system. In these embodiments, the sustained release delivery system includes about 18% locust bean gum; about 12 % xanthan gum; about 60 % mannitol; and about 10 % calcium sulfate dihydrate.

In some embodiments, the present methods employ oral sustained release solid dosage formulations including about 60 mg nalbuphine hydrochloride, and from about 25 mg to about 225 mg, for example about 120 mg, of a sustained release delivery system. In these embodiments, the sustained release delivery system includes about 10% locust bean gum; about 12 % xanthan gum; about 60% mannitol; and about 10% calcium sulfate dihydrate. In some embodiments, the present methods employ oral sustained release solid dosage formulations including from about 5 mg to about 80 mg nalbuphine hydrochloride and about 80 mg to about 360 mg of a sustained release delivery system.

In some embodiments, the present methods employ oral sustained release solid dosage formulations including about 120 mg nalbuphine hydrochloride, and from about 25 mg to about 250 mg, for example about 240 mg, of a sustained release delivery system. In these embodiments, the sustained release delivery system includes about 18% locust bean gum; about 12 % xanthan gum; about 60 % mannitol; and about 10 % calcium sulfate dihydrate.

In some embodiments, the present methods employ oral sustained release solid dosage formulations including about 30 mg nalbuphine hydrochloride, and from about 25 mg to about 350 mg, for example about 270 mg or about 360 mg, of a sustained release delivery system. In these embodiments, the sustained release delivery system includes about 18% locust bean gum; about 12 % xanthan gum; about 60 % mannitol; and about 10 % calcium sulfate dihydrate.

In some embodiments, the present methods employ oral sustained release solid dosage formulations including about 45 to about 60 mg nalbuphine hydrochloride and from about 100 mg to about 200 mg of a sustained release delivery system. In these embodiments, the sustained release delivery system includes about 15% to about 25% locust bean gum; about 10% to about 20% xanthan gum; about 50% to about 85% mannitol; and about 5% to about 15% calcium sulfate dihydrate.

In some embodiments, the present methods employ oral sustained release solid dosage formulations including about 30 mg nalbuphine hydrochloride, about 32.4 mg locust bean gum; about 21.6 mg xanthan gum; about 108 mg mannitol; about 18 mg calcium sulfate dihydrate, about 35 mg hydroxypropylcellulose, and about 1.9 mg magnesium stearate.

In some embodiments, the present methods employ oral sustained release solid dosage formulations including about 60 mg nalbuphine hydrochloride, about 21.6 mg locust bean gum; about 14.4 mg xanthan gum; about 72 mg mannitol; about 12 mg calcium sulfate dihydrate, about 30 mg hydroxypropylcellulose, and about 1.6 mg magnesium stearate.

In some embodiments, the present methods employ oral sustained release solid dosage formulations including about 90 mg nalbuphine hydrochloride, about 32.4 mg locust bean gum; about 21.6 mg xanthan gum; about 108 mg mannitol; about 18 mg calcium sulfate dihydrate, about 45 mg hydroxypropylcellulose, and about 2.4 mg magnesium stearate.

In some embodiments, the present methods employ oral sustained release solid dosage formulations including about 120 mg nalbuphine hydrochloride, about 43.2 mg locust bean gum; about 28.8 mg xanthan gum; about 144 mg mannitol; about 24 mg calcium sulfate dihydrate, about 60 mg hydroxypropylcellulose, and about 3.2 mg magnesium stearate.

In some embodiments, the present methods employ oral sustained release solid dosage formulations including about 180 mg nalbuphine hydrochloride, about 64.8 mg locust bean gum; about 43.2 mg xanthan gum; about 216 mg mannitol; about 36 mg calcium sulfate dihydrate, about 90 mg hydroxypropylcellulose, about 5 mg magnesium stearate, and about 25 mg fumaric acid.

In some embodiments, the present methods employ oral sustained release solid dosage formulations including about 180 mg nalbuphine hydrochloride, about 48.6 mg locust bean gum; about 32.4 mg xanthan gum; about 162 mg mannitol; about 27 mg calcium sulfate dihydrate, about 60 mg hydroxypropylcellulose, about 4 mg magnesium stearate, and about 25 mg fumaric acid.

In some embodiments, the present methods employ oral sustained release solid dosage formulations including about 30 mg nalbuphine hydrochloride, about 32.4 mg locust bean gum; about 21.6 mg xanthan gum; about 108 mg mannitol; about 18 mg calcium sulfate dihydrate, about 35 mg hydroxypropylcellulose, about 1.9 mg magnesium stearate, and about 7.4 mg Opadry II White.

The sustained release formulations of nalbuphine are orally administrable solid dosage formulations. Nonlimiting examples of oral solid dosage formulations include tablets, capsules including a plurality of granules, sublingual tablets, powders, granules, syrups, and buccal dosage forms or devices (e.g., buccal patches, tablets, etc.). In some embodiments, tablets have an enteric coating or a hydrophilic coating.

The sustained release delivery system is prepared by dry granulation or wet granulation, before the nalbuphine or pharmaceutically acceptable salt, solvate or ester thereof is added, although the components can be held together by an agglomeration technique to produce an acceptable product. In the wet granulation technique, the components (e.g., hydrophilic compounds, cross-linking agents, pharmaceutical diluents, cationic cross-linking compounds, hydrophobic polymers, etc.) are mixed together and then moistened with one or more liquids (e.g., water, propylene glycol, glycerol, alcohol) to produce a moistened mass that is subsequently dried. The dried mass is then milled with conventional equipment into granules of the sustained release delivery system. Thereafter, the sustained release delivery system is mixed in the desired amounts with the nalbuphine or the pharmaceutically acceptable salt, solvate or ester thereof and, optionally, one or more wetting agents, one or more lubricants, one or more buffering agents, one or more coloring agents, one or more second hydrophilic compounds, or other conventional ingredients, to produce a granulated composition. The sustained release delivery system and the nalbuphine can be blended with, for example, a high shear mixer. The nalbuphine is preferably finely and homogeneously dispersed in the sustained release delivery system. The granulated composition, in an amount sufficient to make a uniform batch of tablets, is subjected to tableting in a conventional production scale tableting machine at typical compression pressures, i.e., about 2,000-16,000 psi. In some embodiments, the mixture should not be compressed to a point where there is subsequent difficulty with hydration upon exposure to liquids.

In some embodiments, the nalbuphine formulation is prepared by dry granulation or wet granulation. The components of the sustained release delivery system are added, along with the nalbuphine or a pharmaceutically acceptable salt, solvate or ester thereof. Alternatively, all of the components can be held together by an agglomeration technique to produce an acceptable product. In the wet granulation technique, nalbuphine or pharmaceutically salt, solvate or ester thereof and the components (e.g., hydrophilic compounds, cross-linking agents, pharmaceutical diluents, cationic cross-linking compounds, hydrophobic polymers, etc.) are mixed together and then moistened with one or more liquids (e.g., water, propylene glycol, glycerol, alcohol) to produce a moistened mass that is subsequently dried. The dried mass is then milled with conventional equipment into granules. Optionally, one or more wetting agents, one or more lubricants, one or more buffering agents, one or more coloring agents, one or more second hydrophilic compounds, or other conventional ingredients, are also added to the granulation. The granulated composition, in an amount sufficient to make a uniform batch of tablets, is subjected to tableting in a conventional production scale tableting machine at typical compression pressures, i.e., about 2,000-16,000 psi. In some embodiments, the mixture should not be compressed to a point where there is subsequent difficulty with hydration upon exposure to liquids.

The average particle size of the granulated composition is from about 50 µm to about 400 gm by weight. In some embodiments, the average particle size by weight is from about 185 µm to about 265 µm. The average density of the granulated composition is from about 0.3 g/mL to about 0.8 g/mL. In some embodiments, the average density is from about 0.5 g/mL to about 0.7 g/mL. The tablets formed from the granulations are generally from about 4 Kp to about 22 Kp hardness. The average flow of the granulations is from about 25 to about 40 g/sec.

In some embodiments, the present methods can employ a multilayer solid dosage form, in which the layers are formulated to release the nalbuphine hydrochloride at different rates. For example, in one embodiment, the second layer is an extended release layer that includes nalbuphine or a pharmaceutically acceptable salt, solvate or ester thereof and a sustained release delivery system designed to release the nalbuphine or the pharmaceutically acceptable salt, solvate or ester thereof at a controlled rate so that therapeutically beneficial blood levels are maintained over an extended period of time (e.g., from about 8 to about 12 hours). The first layer is an immediate release layer that includes a formulation of nalbuphine or a pharmaceutically acceptable salt, solvate or ester thereof designed to release the nalbuphine or the pharmaceutically acceptable salt, solvate or ester thereof at a rate that is faster than the rate of the second layer to achieve a therapeutically beneficial blood level in an immediate period of time (e.g., from about 1 to about 2 hours). In some embodiments, the first layer includes a sustained release delivery system. In some embodiments, the first layer does not include a sustained release delivery system.

In some embodiments, the weight ratio of the second layer to the first layer is about 10:1 to about 1:10, about 9:1 to about 1:9, about 8:1 to about 1:8, about 7:1 to about 1:7, about 6:1 to about 1:6, about 5:1 to about 1:5, about 4:1 to about 1:4, about 3:1 to about 1:3, about 2:1 to about 1:2. In one embodiment, the weight ratio of the second layer to the first layer is about 5:1 to about 1:5. In a further embodiment, the weight ratio of the second layer to the first layer is about 1:1 to about 1:2. In some embodiments, the weight ratio of the second layer to the first layer is about 1:1, about 1:1.2, about 1:1.4, about 1:1.6, about 1:1.8, or about 1:2. In one embodiment, the weight ratio of the second layer to the first layer is about 1:2. In one embodiment, the weight ratio of the second layer to the first layer is about 1:1.4. In some embodiments, the weight ratio of the second layer to the first layer is about 3:1, about 2.5:1, about 2:1, about 1.5:1. In one embodiment, the weight ratio of the second layer to the first layer is about 2.5:1.

The sustained release delivery system of the multilayer dosage form includes (i) at least one hydrophilic compound, at least one cross-linking agent, and at least one pharmaceutical diluent; (ii) at least one hydrophilic compound, at least one cross-linking agent, at least one pharmaceutical diluent, and at least one cationic cross-linking agent different from the first cross-linking agent; or (iii) at least one hydrophilic compound, at least one cationic cross-linking compound, and at least one pharmaceutical diluent. In some embodiments, when the first layer includes a sustained release delivery system, the sustained release delivery system of the first layer includes the same components as the sustained release delivery system of the second layer (e.g., both the first and second layers are one of embodiments (i)-(iii), listed above). In other embodiments, the sustained release delivery system of the first layer includes different components as the sustained release delivery system of the second layer (e.g., the first layer is embodiment (i), listed above, while the second layer is embodiment (iii), listed above). It is recognized that the sustained release delivery system of either layer can be one of embodiments (i)-(iii) listed above. Moreover, it is recognized that in some embodiments, the first layer does not include a sustained release delivery system.

The sustained release delivery system is generally present in the second layer (e.g., extended release layer) in an amount ranging from about 10 mg to about 420 mg. In some embodiments, the sustained release delivery system is present in the second layer in an amount ranging from about 110 mg to about 200 mg. In some embodiments, the sustained release delivery system is present in the second layer in an amount ranging from about 110 mg to about 150 mg. In some embodiments, the sustained release delivery system is present in the second layer in an amount ranging from about 90 mg to about 150 mg. In some embodiments, the sustained release delivery system is present in the second layer in an amount of about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, or about 200 mg. In one embodiment, the sustained release delivery system is present in the second layer in an amount of about 123 mg. In one embodiment, the sustained release delivery system is present in the second layer in an amount of about 101 mg. In one embodiment, the sustained release delivery system is present in the second layer in an amount of about 92 mg. In another embodiment, the sustained release delivery system is present in the second layer in an amount of about 112.5 mg. In one embodiment, the sustained release delivery system is present in the second layer in an amount of about 135 mg. In one embodiment, the sustained release delivery system is present in the second layer in an amount of about 150 mg.

Nalbuphine or a pharmaceutically acceptable salt, solvate or ester thereof is generally present in the second layer in an amount ranging from about 15 mg to about 60 mg. In some embodiments, nalbuphine or a pharmaceutically acceptable salt, solvate or ester thereof is present in the second layer in an amount ranging from about 30 mg to about 60 mg. In some embodiments, nalbuphine or a pharmaceutically acceptable salt, solvate or ester thereof is present in the second layer in an amount ranging from about 45 mg to about 60 mg. In one embodiment, nalbuphine or a pharmaceutically acceptable salt, solvate or ester thereof is present in the second layer in an amount of about 15 mg. In one embodiment, nalbuphine or a pharmaceutically acceptable salt, solvate or ester thereof is present in the second layer in an amount of about 30 mg. In one embodiment, nalbuphine or a pharmaceutically acceptable salt, solvate or ester thereof is present in the second layer in an amount of about 45 mg. In one embodiment, nalbuphine or a pharmaceutically acceptable salt, solvate or ester thereof is present in the second layer in an amount of about 15 mg, about 30 mg, about 60 mg, about 90 mg, about 120 mg, or about 180 mg.

In some embodiments, the weight ratio of nalbuphine or a pharmaceutically acceptable salt, solvate or ester thereof to the sustained release delivery system in the second layer is about 10:1 to about 1:10, about 9:1 to about 1:9, about 8:1 to about 1:8, about 7:1 to about 1:7, about 6:1 to about 1:6, about 5:1 to about 1:5, about 4:1 to about 1:4, about 3:1 to about 1:3, or about 2:1 to about 1:2. In one embodiment, the weight ratio of nalbuphine or a pharmaceutically acceptable salt, solvate or ester thereof to the sustained release delivery system in the second layer is about 1:2 to about 1:4. In one embodiment, the weight ratio of nalbuphine or a pharmaceutically acceptable salt, solvate or ester thereof to the sustained release delivery system in the second layer is about 1:1 to about 1:5. In some embodiments, the weight ratio of nalbuphine or a pharmaceutically acceptable salt, solvate or ester thereof to the sustained release delivery system in the second layer is about 1: 1, about 1:1.2, about 1:1.4, about 1:1.6, about 1:1.8, about 1:2, about 1:2.5, about 1:3, or about 1:3.5. In one embodiment, the weight ratio of nalbuphine or a pharmaceutically acceptable salt, solvate or ester thereof to the sustained release delivery system in the second layer is about 1:2.5. In another embodiment, the weight ratio of nalbuphine or a pharmaceutically acceptable salt, solvate or ester thereof to the sustained release delivery system in the second layer is about 1:3.3. In a further embodiment, the weight ratio of nalbuphine or a pharmaceutically acceptable salt, solvate or ester thereof to the sustained release delivery system in the second layer is about 1:3. In yet another embodiment, the ratio of nalbuphine or a pharmaceutically acceptable salt, solvate or ester thereof to the sustained release delivery system in the second layer is about 1:2.

When the sustained release delivery system is present in the first layer (e.g., immediate release layer), it is generally present in an amount ranging from about 0 mg to about 50 mg. In some embodiments, the sustained release delivery system is present in the first layer in an amount ranging from about 5 mg to about 25 mg or from about 5 mg to about 15 mg. In one embodiment, the sustained release delivery system is present in the first layer in an amount of about 3 mg to about 9 mg. In one embodiment, the sustained release delivery system is present in the first layer in an amount of about 4 mg to about 6 mg. In some embodiments, the sustained release delivery system is present in the first layer in an amount of about 2 mg, about 4 mg, about 6 mg, about 8 mg, about 10 mg, about 12 mg, about 14 mg, about 15 mg, about 16 mg, about 18 mg, about 20 mg about 25 mg, about 30 mg, about 35 mg, about 40 mg, about 45 mg or about 50 mg. In one embodiment, the sustained release delivery system is present in the first layer in an amount of about 6 mg.

In some embodiments, nalbuphine or a pharmaceutically acceptable salt, solvate or ester thereof is generally present in the first layer (e.g., immediate release layer) in an amount ranging from about 5 mg to about 180 mg. In some embodiments, nalbuphine or a pharmaceutically acceptable salt, solvate or ester thereof is present in the first layer in an amount ranging from about 5 mg to about 25 mg or from about 10 mg to about 20 mg. In some embodiments, the nalbuphine or a pharmaceutically acceptable salt, solvate or ester thereof is present in the first layer in an amount of about 5 mg, about 10 mg, about 11 mg, about 12 mg, about 13 mg, about 14 mg, about 15 mg, about 16 mg, about 17 mg, about 18 mg, about 19 mg, about 20 mg, about 25 mg, about 30 mg, about 35 mg, about 40 mg, about 45 mg or about 50 mg. In one embodiment, nalbuphine or a pharmaceutically acceptable salt, solvate or ester thereof is present in the first layer in an amount of about 15 mg, about 30 mg, about 60 mg, about 90 mg, about 120 mg, or about 180 mg.

In some embodiments, when the first layer includes a sustained release delivery system, the ratio of nalbuphine or pharmaceutically acceptable salt, solvate or ester thereof to the sustained release delivery system in the first layer is about 10:1 to about 1:10, about 9:1 to about 1:9, about 8:1 to about 1:8, about 7:1 to about 1:7, about 6:1 to about 1:6, about 5:1 to about 1:5, about 4:1 to about 1:4, about 3:1 to about 1:3, about 2:1 to about 1:2. In one embodiment, the ratio of nalbuphine or pharmaceutically acceptable salt, solvate or ester thereof to the sustained release delivery system in the first layer is about 2:1 to about 4:1. In some embodiments, the ratio of nalbuphine or pharmaceutically acceptable salt, solvate or ester thereof to the sustained release delivery system in the first layer is about 5:1, about 4.5:1, about 4:1, about 3.5:1, about 3:1, about 2.5:1, about 2:1, about 1.5:1, or about 1:1. In one embodiment, the ratio of nalbuphine or pharmaceutically acceptable salt, solvate or ester thereof to the sustained release delivery system in the first layer is about 2.5:1. In another embodiment, the ratio of nalbuphine or pharmaceutically acceptable salt, solvate or ester thereof to the sustained release delivery system in the first layer is about 3:1.

In some embodiments, the multilayer dosage form further includes a pharmaceutical disintegrant. The disintegrant promotes the dissolution and absorption of nalbuphine or pharmaceutically acceptable salt, solvate or ester thereof from the immediate release layer. Nonlimiting examples of pharmaceutical disintegrants include croscarmellose sodium, starch glycolate, crospovidone, and unmodified starch. In one embodiment, the disintegrant is in the first layer (i.e., the immediate release layer), of the dosage form. The disintegrant is generally present in the layer in an amount of about 1.5 mg to about 4.5 mg. In one embodiment, the disintegrant is present in an amount of about 3 mg. In one embodiment, the disintegrant is present in the layer in an amount of about 2-10% by weight. In one embodiment, the disintegrant is present in the layer in an amount of about 5% by weight. When the layer contains a sustained release delivery system, the weight ratio of the sustained release delivery system to the disintegrant is in a range of about 5:1 to about 1:5. In some embodiments, the ratio of the sustained release delivery system to the disintegrant is in a range of about 1:1 to about 3:1. In other embodiments, the ratio of the sustained release delivery system to the disintegrant is in a range of about 2:1.

In some embodiments, the multilayer tablets are prepared by first preparing the immediate release layer and extended release layer blends separately. The extended release layer is prepared as described above. The wet granulation of the extended release layer is then dried and milled to an appropriate size. Magnesium stearate is added and mixed with the milled granulation. The immediate release layer is prepared by first mixing the nalbuphine or the pharmaceutically acceptable salt, solvate or ester thereof with one or more diluents (e.g., microcrystalline cellulose). This mix is then optionally mixed with one or more disintegrants. The blend is mixed with magnesium stearate. Finally, the immediate release layer blend and the extended release layer blend are compressed into multi-layer (e.g., bi-layer) tablets.

In certain embodiments, the chemistry of certain of the components of the formulation, such as the hydrophilic compound (e.g., xanthan gum), is such that the components are considered to be self-buffering agents which are substantially insensitive to the solubility of the nalbuphine and the pH changes along the length of the gastrointestinal tract. Moreover, the chemistry of the components is believed to be similar to certain known muco-adhesive substances, such as polycarbophil. Muco-adhesive properties are desirable for buccal delivery systems. Thus, the sustained release formulation can loosely interact with the mucin in the gastrointestinal tract and thereby provide another mode by which a constant rate of delivery of the nalbuphine is achieved.

The two phenomenon discussed above (buoyancy and muco-adhesive properties) are mechanisms by which the sustained release formulations can interact with the mucin and fluids of the gastrointestinal tract and provide a constant rate of delivery of the nalbuphine.

When measured by USP Procedure Drug Release General Chapter <711> Dissolution, (incorporated by reference herein in its entirety), the sustained release formulations employed in the present methods generally exhibit an in vitro dissolution of about 15% to about 50% by weight nalbuphine after 1 hour, about 45% to about 80% by weight nalbuphine after 4 hours, or at least about 80% by weight nalbuphine after 10 hours. In some embodiments, the in vitro and in vivo release characteristics of the sustained release formulations are modified using mixtures of one or more different water insoluble and/or water soluble compounds, using different plasticizers, varying the thickness of the sustained release film, including providing release-modifying compounds in the coating, and/or by providing passageways through the coating. In some embodiments, the dissolution rate is determined using apparatus USP Type III/250 mL at pH 6.8, 37° C. and 15 dpm. In some embodiments, the dissolution rate is determined using apparatus USP Type III/250 mL performed in pH change (0-1 hours pH 1.2, after hour 1 pH 4.5, after hour 2 pH 6.8) at 37° C. and 15 dpm.

In some embodiments, the sustained release formulation has an in vitro dissolution of about 50% to about 100% by weight nalbuphine after about 6 hours. In some embodiments, the sustained release formulation has an in vitro dissolution of about 75% to about 100% by weight nalbuphine after about 6 hours. In other embodiments, the sustained release formulation has an in vitro dissolution of about 75% to about 100% by weight nalbuphine from about 6 hours to about 8 hours. In further embodiments, the sustained release formulation has an in vitro dissolution of about 80% to about 100% by weight nalbuphine after about 12 hours. In still other embodiments, the sustained release formulation has an in vitro dissolution of about 80% to about 100% by weight nalbuphine from about 12 hours to about 24 hours. In some embodiments, the sustained release formulation has an in vitro dissolution of about 80% to about 100% after about 8 hours to about 12 hours. In yet other embodiments, the sustained release formulation has an in vitro dissolution of about 15% to about 75% by weight nalbuphine after about 1 hour. In still further embodiments, the sustained release formulation has an in vitro dissolution of about 50% by weight nalbuphine after about 1 hour. In some embodiments, the sustained release formulation has an in vitro dissolution of about 50% by weight nalbuphine after about 1 hour and about 75% to about 100% by weight nalbuphine from about 6 hours to about 8 hours. In some embodiments, the sustained release formulation has an in vitro dissolution of about 50% by weight nalbuphine after about 1 hour and about 75% to about 100% by weight nalbuphine from about 8 hours to about 12 hours. In some embodiments, the sustained release formulation has an in vitro dissolution of about 50% by weight nalbuphine after about 1 hour and about 75% to about 100% by weight nalbuphine from about 12 hours to about 24 hours. In some embodiments, the sustained release formulation has an in vitro dissolution of about 50% by weight nalbuphine after about 1 hour and about 80% to about 100% by weight nalbuphine after about 12 hours.

Where the tablet is a multilayer dosage form having a first extended release layer and a second, immediate release, layer, the sustained release formulation has an in vitro dissolution of about 25% to about 75% by weight nalbuphine after about 1 hour. In some embodiments, the multilayer dosage form has an in vitro dissolution of about 25% by weight nalbuphine after about 1 hour. In some embodiments, the multilayer dosage form has an in vitro dissolution of about 50% by weight nalbuphine after about 1 hour. In some embodiments, the multilayer dosage form has an in vitro dissolution of about 75% to about 100% nalbuphine after about 6-8 hours. In some embodiments, the multilayer dosage form has an in vitro dissolution of about 75% to about 100% nalbuphine after about 8-12 hours. In some embodiments, the multilayer dosage form has an in vitro dissolution of about 75% to about 100% nalbuphine after about 12-24 hours. In some embodiments, the multilayer dosage form has an in vitro dissolution of about 75% to about 100% nalbuphine after about 12 hours.

In some embodiments, when administered orally to patients having either normal or impaired (e.g., reduced) kidney function, the sustained release formulations described herein exhibit the following in vivo characteristics: (a) a peak plasma level of nalbuphine occurs within about 4 hours to about 6 hours, e.g., for patients with uremic pruritus or renal impairment, or about 3 hours to about 5 hours, e.g., for patients without renal impairment after administration; (b) onset of nalbuphine anti-pruritic effect from about 30 minutes of dosing to within about 6 hours of dosing; (c) duration of the nalbuphine anti-pruritic effect is about 2 to about 24 hours; and (d) the relative nalbuphine bioavailability is about 0.5, about 1, about 1.5 or between about 0.5 to about 1.5 compared to an orally administered aqueous solution of nalbuphine. The time of onset for an anti-pruritic effect can depend on at least on dosing and the severity of pruritic symptoms. In some embodiments, the duration of the nalbuphine anti-pruritic effect is at least about 8 hours. In some embodiments, the duration of the nalbuphine anti-pruritic effect is at least about 9 hours. In some embodiments, the duration of the nalbuphine anti-pruritic effect is at least about 10 hours. In some embodiments, the duration of the nalbuphine anti-pruritic effect is at least about 11 hours. In some embodiments, the duration of the nalbuphine anti-pruritic effect is at least about 12 hours. In some embodiments, the duration of nalbuphine anti-pruritic effect is about 6, hours, 8 hours, 10 hours, 12 hours, 15 hours, or 18 hours. In some embodiments, the relative nalbuphine bioavailability is about 0.94 compared to an orally administered aqueous solution of nalbuphine. In some embodiments, the relative nalbuphine bioavailability is about 1.35 compared to an orally administered aqueous solution of nalbuphine.

In some embodiments, the sustained release nalbuphine formulations provide an oral unit dosage form including nalbuphine or a pharmaceutically acceptable salt, solvate or ester thereof. The oral dosage form provides an anti-pruritic effect over a period of at least about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours or about 24 hours. In some embodiments, the oral dosage form provides an anti-pruritic effect over a period of about 6-18 hours, about 8-16 hours, about 8-12 hours, about 8 to about 24 hours, about 12 to about 24 hours, about 18 to about 24 hours, or about 8-10 hours. The oral dosage form provides an anti-pruritic effect over a period of about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours or about 24 hours.

In one embodiment, the oral dosage form provides an anti-pruritic effect as well as breaking the cycle effect, e.g., the itchy sensation does not return after certain treatment period.

In some embodiments, the oral dosage form provides a blood plasma level of nalbuphine characterized by one or more peaks followed by a plateau region. The plateau region is characterized as having a relatively consistent blood plasma level of nalbuphine (e.g., the blood plasma level of nalbuphine does not consistently increase or decrease from time point to time point). In some embodiments, the plateau region is characterized as having a consistent average blood plasma level of nalbuphine. The plateau region is contrasted with the region following the plateau region, in which the blood plasma level of nalbuphine generally decreases from one time point to the next. In some embodiments, the plateau region has a duration of at least about 1 hour, about 2 hours, about 3 hours, about 4 hours, about 5 hours, about 6 hours, about 7 hours, about 8 hours, about 9 hours, about 10 hours, about 11 hours or about 12 hours. In some embodiments, the plateau region has a duration from about 1 hour to about 12 hours, from about 2 hours to about 10 hours, from about 2 hours to about 8 hours, from about 2 hours to about 7 hours or from about 4 hours to about 10 hours, from about 4 hours to about 8 hours, or from about 4 hours to about 6 hours. In some embodiments, the blood plasma level of nalbuphine at each time point in the plateau region ranges from about 75% to about 125% of the mean blood plasma level in the plateau region. In some embodiments, the blood plasma level of nalbuphine at each time point in the plateau region ranges from about 80% to about 120% of the mean blood plasma level in the plateau region. In some embodiments, the blood plasma level of nalbuphine at each time point in the plateau region ranges from about 85% to about 115% of the mean blood plasma level in the plateau region. In some embodiments, the blood plasma level of nalbuphine at each time point in the plateau region ranges from about 90% to about 110% of the mean blood plasma level in the plateau region.

In some embodiments, the minimum blood plasma level of nalbuphine observed during the plateau region is not more than about 25% below the mean blood plasma level for all time points in the plateau region. In some embodiments, the minimum blood plasma level of nalbuphine observed during the plateau region is not more than about 20% below the mean blood plasma level in the plateau region. In some embodiments, the minimum blood plasma level of nalbuphine observed during the plateau region is not more than about 15% below the mean blood plasma level in the plateau region. In some embodiments, the minimum blood plasma level of nalbuphine observed during the plateau region ranges from about 75% to about 100% of the mean blood plasma level in the plateau region. In some embodiments, the minimum blood plasma level of nalbuphine observed during the plateau region ranges from about 80% to about 100% of the mean blood plasma level in the plateau region. In some embodiments, the minimum blood plasma level of nalbuphine observed during the plateau region ranges from about 85% to about 100% of the mean blood plasma level in the plateau region. In some embodiments, the minimum blood plasma level of nalbuphine observed during the plateau region ranges from about 80% to about 95% of the mean blood plasma level in the plateau region.

### Co-Therapy

While the compositions can be administered as the sole active pharmaceutical ingredient or sole active anti-pruritus ingredient in the methods described herein, in other embodiments they can also be used in combination with one or more ingredients which are known to be therapeutically effective against pruritus and/or compliment the effect of anti-pruritus ingredient.

For example, in some embodiments, the present methods can employ nalbuphine or a pharmaceutically acceptable salt, solvate or ester thereof in conjunction with one or more anti-pruritic agents. In some embodiments, additional compounds combined with the anti-pruritic agent, e.g., nalbuphine, or a pharmaceutically acceptable salt, solvate or ester thereof, include antihistamines, anti-inflammatory corticosteroids, topical anti-infectives and antifungals, antibacterials, and antivirals, cytotoxic agents, and counterirritants/analgesics. Other antipruritic agents include anti-depressants, vitamin D, kappa agonists, irritants such as coal tar derivatives and psoralens, 5-HT3 antagonists such as ondansetron, H2 receptor antagonist such as cimetidine, H1 receptor antagonist such as cetirizine, immunomodulators such as tacrolimus, immunosuppressants such as cyclosporine A, µ- antagonists, capsaicin, cannabinoids, latex extracts from various Croton species found in the Amazon jungle (e.g., Zangrado^{®}), or Nk1 antagonists, etc. In some embodiments, nalbuphine or a pharmaceutically acceptable salt, solvate or ester thereof is not administered in combination with a second anti-pruritus agent, e.g., co-formulated or administered separately.

### Dosing

The invention provides methods for treating pruritus by administering an effective amount of an anti-pruritic agent, i.e., nalbuphine or a pharmaceutically acceptable salt, solvate or ester thereof, to a patient in need thereof. An effective amount is an amount sufficient to eliminate or significantly reduce pruritus symptoms or to alleviate those symptoms (e.g., reduce the symptoms, such as itching, compared to the symptoms present prior to treatment). Formulations employed in the present methods can incorporate the anti-pruritic agent in a sustained release formulation such that the formulation provides therapeutically effective blood plasma levels of nalbuphine for the treatment of pruritus.

According to some embodiments of the present invention, the methods of the present invention provide therapeutically effective blood plasma levels of nalbuphine for treating uremic pruritus. Blood plasma levels of nalbuphine may be expressed using pharmacokinetic parameters that are known to those skilled in the art, such as steady state plasma levels, AUC, Cmax and Cmin. In some embodiments, the present methods provide steady state plasma levels of nalbuphine that correlate to one or more statistically significant therapeutic effects. In certain embodiments, the therapeutically effective steady state plasma levels of nalbuphine provided by the methods of the present invention range from about 20 ng/mL to about 80 ng/mL, including about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 65 ng/mL, about 70 ng/mL, about 75 ng/mL and about 80 ng/mL, including all ranges there between.

According to some embodiments of the present invention, administering of nalbuphine or a pharmaceutically acceptable salt, solvate or ester thereof provides statistically significant therapeutic effect. In one embodiment, the statistically significant therapeutic effect is determined based on one or more standards or criteria provided by one or more regulatory agencies in the United States, e.g., FDA or other countries. In another embodiment, the statistically significant therapeutic effect is determined based on results obtained from regulatory agency approved clinical trial set up and/or procedure.

In some embodiments, the statistically significant therapeutic effect is determined based on a patient population of at least 100, 200, 300, 400, 500, 600, 700, 800, 900, 1000 or 2000. In some embodiments, the statistically significant therapeutic effect is determined based on data obtained from randomized and double blinded clinical trial set up. In some embodiments, the statistically significant therapeutic effect is determined based on data with a p value of less than or equal to about 0.05, 0.04, 0.03, 0.02 or 0.01. In some embodiments, the statistically significant therapeutic effect is determined based on data with a confidence interval greater than or equal to 95%, 96%, 97%, 98% or 99%. In some embodiments, the statistically significant therapeutic effect is determined on approval of Phase III clinical trial of the methods provided by the present invention, e.g., by FDA in the US.

In some embodiments, the statistically significant therapeutic effect is determined by a randomized double blind clinical trial of patients treated with nalbuphine or a pharmaceutically acceptable salt, solvate or ester thereof and optionally in combination with standard care. In some embodiment, the statistically significant therapeutic effect is determined by a randomized clinical trial and using Numerical Rating Scale (NRS) as primary efficacy parameter and optionally in combination with any other commonly accepted criteria for pruritus assessment.

In general, statistical analysis can include any suitable method permitted by a regulatory agency, e.g., FDA in the US or Europe or any other country. In some embodiments, statistical analysis includes non-stratified analysis, log-rank analysis, e.g., from Kaplan-Meier, Jacobson-Truax, Gulliken-Lord-Novick, Edwards-Nunnally, Hageman-Arrindel and Hierarchical Linear Modeling (HLM) and Cox regression analysis.

According to the present invention, the anti-pruritic agent is administered on a once or twice a day basis to provide effective relief of the symptoms of uremic pruritus. The total daily dose is 240 mg. In some embodiments, the total daily dose of the anti-pruritic agent is 240 mg a day for the treatment of uremic pruritus.

In some embodiments, 120 mg of the anti-pruritus agent twice a day is selected to provide a substantial reduction in itch for patients with uremic pruritus. In some embodiments, 240 mg of the anti-pruritus agent once a day is selected to provide a substantial reduction in itch for patients with uremic pruritus.

Reduction of itch in patients with pruritic conditions can be determined by various methods. In some embodiments, the effectiveness of a dosage regimen can be determined by evaluation via a Pruritus Visual Analog Scale (VAS) test. In some embodiments, the effectiveness of a dosage regimen can be determined by evaluation via a worst or average itching intensity Numerical Rating Scale (NRS). In yet some other embodiments, the effectiveness of a dosage regimen can be determined by evaluation via a worst or average itching intensity Numerical Rating Scale (NRS), an Itch Medical Outcomes Study (MOS) Sleep scale, a Skindex-10, a Hospital Anxiety and Depression Scale (HADS), a Global Physician index scale, Patient Benefit Index - pruritus version (PBI-P), Prurigo Activity Score (PAS), itchy, burning and stinging Verbal Rating Scale (VRS) score, Itchy Quality of Life (ItchyQoL), a Patient Assessed Disease Severity Scale, Patient Global Assessment (PGA) via ItchApp, vPGA, Dermatology Life Quality Index (DLQI), Nocturnal scratching using actigraphy, nerve fiber density and MOR/KOR density, Brief Itching Inventory, Beck Depression Index, or any combination thereof. In still another embodiment, the effectiveness of a dosage regimen can be determined by evaluation via a worst or average itching intensity NRS as a primary efficacy endpoint in association with secondary efficacy endpoints such as an Itch Medical Outcomes Study (MOS) Sleep scale, a Skindex-10, a Hospital Anxiety and Depression Scale (HADS), a Global Physician index scale, Patient Benefit Index - pruritus version (PBI-P), Prurigo Activity Score (PAS), itchy, burning and stinging Verbal Rating Scale (VRS) score, ItchyQoL, a Patient Assessed Disease Severity Scale, Patient Global Assessment (PGA) via ItchApp, vPGA, Dermatology Life Quality Index (DLQI), Nocturnal scratching using actigraphy, nerve fiber density and MOR/KOR density, Brief Itching Inventory, Beck Depression Index or any combination thereof.

According to some embodiments of the present invention, the dosing frequency and dose amount per administration of the anti-pruritus agent are selected to provide therapeutic effects for the treatment of pruritus. In certain embodiments, nalbuphine or a pharmaceutically acceptable salt, solvate or ester thereof is administered on a once-a-day or twice-a-day basis for at least a week, for example, about a week, about 2 weeks, about 3 weeks, about 4 weeks, about 5 weeks, about 6 weeks, about 7 weeks, about 8 weeks, about 9 weeks, about 10 weeks, about 12 weeks, about 24 weeks, and about 50 weeks. In certain embodiments, 120 mg of nalbuphine or a pharmaceutically acceptable salt, solvate or ester thereof is administered on a twice-a-day basis for at least a week. In certain embodiments, 240 mg of nalbuphine or a pharmaceutically acceptable salt, solvate or ester thereof is administered on a once-a-day basis for at least a week.

In some embodiments, after the treatment the patient experiences a substantial reduction of itch that is characterized by at least about a 30% decline in worst or average itching intensity Numerical Rating Scale (NRS) value compared to prior to the treatment. In some embodiments, the reduction of itch is characterized by a decline in NRS value ranging from about 30% to about 100%, for example, about 30%, about 40%, about 50%, about 60%, about 70%, about 80%, about 90%, and about 100%, compared to prior to the treatment.

In some embodiments, after the treatment the patient experiences a substantial reduction of itch that is characterized by at least about a 10% improvement in total score and/or a subscale score of Skindex-10 compared to prior to the treatment. In some embodiments, the reduction of itch is characterized by an improvement in Skindex-10 score ranging from about 10% to about 100%, for example, about 10%, about 20%, about 30%, about 40%, about 50%, about 60%, about 70%, about 80%, about 90%, and about 100%, compared to prior to the treatment. In some embodiments, the reduction of itch is characterized by an improvement in Skindex-10 disease domain score, Skindex-10 mood/emotional distress domain score, or Skindex-10 social functioning domain score ranging from about 10% to about 100%, for example, about 10%, about 20%, about 30%, about 40%, about 50%, about 60%, about 70%, about 80%, about 90%, and about 100%, compared to prior to the treatment.

In some embodiments, after the treatment the patient experiences a substantial reduction of itch that is characterized by at least about a 20% improvement in Itch Medical Outcomes Study (MOS) sleep scale compared to prior to the treatment. In some embodiments, the reduction of itch is characterized by an improvement in Itch Medical Outcomes Study (MOS) sleep scale ranging from about 10% to about 100%, for example, about 10%, about 20%, about 30%, about 40%, about 50%, about 60%, about 70%, about 80%, about 90%, and about 100%, compared to prior to the treatment.

In some embodiments, the daily dose of the anti-pruritic agent is in a once or twice daily dose, and then titrated upward until the patient experiences satisfactory relief from the pruritic condition. The daily dose can be titrated in increments ranging from about 5 mg to about 240 mg (e.g., about 15 mg, about 30 mg or about 60 mg). The daily dose can be titrated in one or more steps. The daily dosage can be titrated by increasing a single daily dosage, or each dose of a twice-daily dosing regimen. The amount a dosage is stepped, where there are multiple titration steps, can be the same, or can be different.

In some embodiments, the titration may be initiated with about 15 mg, about 30 mg or about 60 mg of the anti-pruritic agent once or twice daily. In certain embodiments, doses can be adjusted in 30 mg increments every 1 to 4 days. Patients can self-titrate to effect over from about 7 days to about 30 days (for example, from about 12 days to about 20 days) to a dose that provides adequate relief from itch and minimizes adverse reactions. In some embodiments, the titration is conducted for at least 2 weeks, 3 weeks, 4 weeks or 5 weeks prior to the administration.

In certain embodiments, patients can be provided initially with 15 mg, 30 mg, 60 mg or 90 mg tablets to self-titrate to effect up to about 60 mg, about 90 mg, about 120 mg, about 180 mg, about 240 mg, once or twice a day. In one embodiment, the titration dose is started with about 15 mg or about 30 mg, and then gradually increased to about 60 mg or 120 mg twice a day, e.g., for patients with uremic pruritus. In another embodiment, the titration dose is started with about 15 mg or about 30 mg, and then gradually increased to about 120 mg or 240 mg once a day, e.g., for patients with uremic pruritus.

Opioids (such as nalbuphine) are commonly used to treat acute to severe pain. It is known that patients treated with opioids usually rapidly develop profound tolerance to the analgesic effects, such that dose escalation is required to maintain analgesic efficacy. In contrast, the present disclosure provides methods for treating uremic pruritus by administering an effective amount of nalbuphine or a pharmaceutically acceptable salt, solvate or ester thereof, to a patient in need thereof, wherein the treatment does not require dose escalation during an extended period of administration (e.g., over a period of months of dosing), i.e., the patient does not develop a tolerance to the anti-pruritic effects of nalbuphine.

In some embodiments, the present method of treating uremic pruritus comprises administering for at least a week to a patient in need thereof a daily dose of at least about 60 mg of nalbuphine or a pharmaceutically acceptable salt, solvate or ester thereof, wherein the daily dose of nalbuphine or a pharmaceutically acceptable salt or ester thereof is substantially the same during the administering. According to the invention, the method comprises administering a daily dose of 240 mg of nalbuphine. In particular embodiments, 120 mg of the anti-pruritus agent is administered twice a day. In further embodiments, the administering is for about 12 weeks, 24 weeks or 50 weeks.

According to the invention, the anti-pruritus agent is nalbuphine, and in a non-claimed aspect, the metabolites include glucuronides (most likely on the phenol and cyclohexane rings), two hydroxylated nalbuphine metabolites (on the cyclobutane ring) and three ketones (hydroxylation of the cyclobutane ring, followed by oxidation to a carbonyl or followed by ring opening of the cyclobutane ring). In some embodiments, the nalbuphine metabolites include nalbuphine 3-glucuronide or 6-glucuronide. In some other embodiments, the nalbuphine metabolites include triple hydroxylated nalbuphine, mono-hydroxylated nalbuphine, or mono-glucuronidated nalbuphine or a combination thereof. In certain embodiments, the one or more metabolites of the anti-pruritus agent do not have detectable anti-pruritus activity. In other embodiments, one or more of the metabolites of the anti-pruritus agent exhibit anti-pruritus activity.

In embodiments wherein one or more metabolites of the anti-pruritus agent exhibit anti-pruritus activity, the dosing regimen of the anti-pruritus agent may be adjusted and/or titrated as described hereinabove depending on the clearance rate of the one or more metabolites exhibiting anti-pruritic activity. Such dosage adjustment and/or titration of the dosage of the anti-pruritic agent can be performed to prevent accumulation of either the anti-pruritic agent and/or one or more metabolites, which can also exhibit anti-pruritic activity, to avoid toxicity effects in a patient treated with the present anti-pruritic agent.

In some embodiments, the anti-pruritus agent is completely metabolized (e.g., about 100% metabolized). In other embodiments, the anti-pruritus agent is not completely metabolized (e.g., less than about 100% metabolized). For example, in some embodiments, the anti-pruritus agent is about 100% metabolized, about 95% metabolized, about 90% metabolized, about 85% metabolized, about 80% metabolized, about 75% metabolized, about 70% metabolized, about 65% metabolized, about 60% metabolized, about 55% metabolized, about 50% metabolized, about 45% metabolized, about 40% metabolized, about 35% metabolized, about 25% metabolized, about 20% metabolized, about 15% metabolized, about 10% metabolized, about 5% metabolized, about 1% metabolized, or about 0% metabolized. In certain embodiments, the amount of dialyzable agent can be measured or monitored by the level of accumulation, e.g., blood plasma level of the anti-pruritus agent or one or more of its metabolites.

The following non-limiting examples illustrate various aspects of the present invention.

### EXAMPLES

### Example 1

A 30 mg or 60 mg extended release (ER) nalbuphine tablet was prepared as follows: Nalbuphine HC1, mannitol, xanthan gum, locust bean gum and calcium sulfate dihydrate were added to a high shear mixer and dried mix at low speed. A granulating solution (water for injection or purified water) was introduced into the mixer at low speed. The wet granulation was granulated at high speed and dried in a fluid bed processor. The dried granules were milled and sized using a conventional mill. The milled granulation was transferred into a diffusion (tumble) mixer. Hydroxypropylcellulose and, when applicable, fumaric acid (180 mg formulations only) were added to the diffusion mixer and blended. Thereafter, magnesium stearate was added to the diffusion mixer and blended. The final blend was compressed using a rotary tablet press. Tablets may be coated with a non-functional Opadry white coating.

**Table 1**

| 30 mg Extended Release Nalbuphine Tablet | |
|---|---|
| **Ingredient** | **mg/tablet** |
| Nalbuphine HCI | 30.0 |
| Mannitol | 108.0 |
| Hydroxypropylcellulose | 35.0 |
| Locust bean gum | 32.4 |
| Xanthan gum | 21.6 |
| Calcium sulfate dehydrate | 18.0 |
| Magnesium stearate | 1.9 |
| Water for injection or Purified water | QS |
| **Total:** | 246.9 |

The tablets were coated with a non-functional coat (Table 2).

**Table 2**

| Nalbuphine HCl ER Tablets, 30 mg or 60 mg Composition | |
|---|---|
| **Component** | **Tablet (mg/tablet)** |
| Nalbuphine HCl | 30.0 |
| Mannitol | 108.0 |
| Hydroxypropylcellulose | 35.0 |
| Locust bean gum | 32.4 |
| Xanthan gum | 21.6 |
| Calcium sulfate dihydrate | 18.0 |
| Magnesium stearate¹ | 1.9 |
| Opadry II White | 7.4 |
| Sterile water for irrigation² | QS |
| **Total** | 254.3 |
| | |

| **Component** | **Tablet (mg/tablet)** |
|---|---|
| Nalbuphine HCl | 60.0 |
| Mannitol | 72.0 |
| Hydroxypropylcellulose | 30.0 |
| Locust bean gum | 21.6 |
| Xanthan gum | 14.4 |
| Calcium sulfate dihydrate | 12.0 |
| Magnesium stearate¹ | 1.6 |
| Opadry II White | 6.355 |
| Sterile water for irrigation² | QS |
| **Total** | 218 |

### Example 2

This clinical study was a parallel, double-blind, placebo-controlled trial in which renally impaired patients on hemodialysis with moderate or severe uremic pruritus were randomized in a 1:1:1 ratio to nalbuphine ER tablets to a target dose of 120 mg BID or 60 mg BID, or matching placebo tablets BID. Medications taken for treatment of pruritus ("antipruritic medications") were not prohibited, but their use was recorded throughout the study. Use of skin emollients was also not restricted. A placebo comparator was chosen because there are no approved treatments for uremic pruritus in the United States or Europe or an established standard of care that could serve as an active control.

The primary objectives were to evaluate the effects on itching intensity using a Worst Itching Intensity Numerical Rating Scale (NRS, 0 [no itching] - 10 [worst possible itching]) as well as safety and tolerability. The study was conducted at 46 United States investigative sites and 6 investigative sites in Romania and Poland. The Sponsor oversaw the conduct of the trial and an independent unblinded Data Safety Monitoring Board reviewed safety data approximately once a month during the conduct of the trial.

### Participants

To be eligible, patients had to have been on hemodialysis for ≥ 3 months, have a Patient Assessed Disease Severity (PADS) category of "B" or "C", and a mean of 6 Worst Itching Numerical Rating Scale Scores during the week prior to randomization ≥4.5 on an 11-point scale (0, "no itching" to 10, "worst possible itching") with at least 2 scores ≥5.0.

### Patient Assessed Disease Severity (PADS)

Which of these patients are you most like? (Mark One)
**Patient A:**
   I do not generally have scratch marks on my skin.
   I do not generally have a problem sleeping because of itching.
   My itching does not generally make me feel agitated or sad.
**Patient B:**
   I sometimes have scratch marks on my skin.
   I sometimes have problems sleeping because of itching.
   My itching can sometimes make me feel agitated or sad.
**Patient C:**
   I often have scratch marks on my skin that may or may not bleed or get infected.
   I often have problems sleeping because of itching.
   My itching often makes me feel agitated or sad.

### Worst Itching Numerical Rating Scale

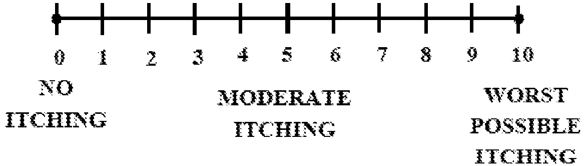

Patients selecting Profile B or C were those who were sometimes or often bothered by scratch marks, problems sleeping because of itching, and feeling sad/agitated because of itching. Additionally, the pruritus could not have been attributed to a condition unrelated to end-stage renal disease such as cholestasis, atopic dermatitis, or lymphoma. All patients gave their written informed consent for study participation using a consent form approved by a central or local institutional review board or ethics committee. The full eligibility criteria can be found below.

### Inclusion Criteria

Patients must meet all of the following criteria to be eligible:
1. Have been adequately informed of the nature and risks of the study and have given written informed consent prior to Screening.
2. Have been receiving in-center hemodialysis for ≥ 3 months and are currently on a schedule of 3 times a week.
3. Have self-categorized themselves as patient types B or C on the Patient Assessed Disease Severity Scale (PADS) at Screening.
4. Have either two measurements of the urea reduction ratio (URR) > 65 or two single-pool Kt/V >1.2 during the three months prior to completing Screening.
5. Have a mean worst itch NRS ≥ 4.5 based on averaging a total of six daytime and nighttime NRS measurements from 3 dialysis visits (3 daytime NRS measurements and 3 nighttime NRS measurements). The measurements will be taken during Week -1 (Visits 4, 5, and 6). If one or two NRS measurements are missed from one visit during Week -1, then the corresponding day or night measurement from Visit 3 may be used to calculate the mean NRS value.
6. Have an absolute minimum worst nighttime or daytime itch ≥ 5 on at least 2 of the six (6) NRS measurements used for satisfying Inclusion Criterion number 5.
7. Are male or female who are at least 18 years old at the time of Screening.
8. Agree to comply with the contraception requirements as below:
   Female patients of childbearing potential are required to use one barrier method (e.g., condom, cervical cap, or diaphragm) of contraception in addition to one other method (e.g., intrauterine device [IUD] in place at least one month, stable hormonal contraception for at least 3 months, tubal ligation, Essure procedure, or spermicide). For female patients using a barrier method plus spermicide, that method must be used for at least 14 days prior to Screening. For the purpose of this study, all females are considered to be of childbearing potential unless they are post-menopausal (i.e., at least 1 year since last menses and age >50 years) or surgically sterile (i.e., tubal ligation, hysterectomy and/or bilateral oophorectomy).

### Exclusion Criteria

If a patient meets any of the following criteria, he or she is not eligible:
1. Have had a significant alteration in dialysis regimen during the Screening Period (i.e., changes in filter type, increase or decrease by > 1 hour per week in prescribed dialysis time, change in type or site of dialysis access, or change in prescribed blood flow rate by > 100 mL/min, etc.). In certain embodiments, "Dialysis regimen" is defined by a dialysis prescription that is used for at least 2 dialysis treatments.
2. Are receiving or anticipated to be receiving nocturnal dialysis or home hemodialysis treatment during the study.
3. Have an alanine aminotransferase (ALT) and/or aspartate aminotransferase (AST) concentration > 3x the upper limit of the normal range (ULN) at Screening.
4. Have a serum total bilirubin > 3x ULN at screening, unless it is explained by a documented history of Gilbert's Disease.
5. Have pruritus that is believed to be caused by a condition unrelated to end-stage renal disease (e.g., cholestasis, atopic dermatitis, lymphoma). Hyperparathyroidism, calcium and phosphate abnormalities, anemia, the uremic milieu, the dialysis procedure and membranes are examples of conditions that are related to end-stage renal disease and are therefore not excluded.
6. In the 2 weeks prior to Screening, has received nalfurafine, naltrexone, or naloxone or is anticipated to receive these drugs during the study.
7. Have a confirmed malignant tumor and receiving active treatment with a systemic drug (hormonal treatment may be acceptable to study enrollment if approved by the medical monitor).
8. Have any significant medical condition or other factors that in the opinion of the Investigator may interfere with the conduct of the study.
9. In the 2 weeks prior to collecting Screening NRS scores, have had any addition or discontinuation of drugs or emollients being taken for pruritus; or any changes in doses of drugs being taken daily for pruritus.
10. Has received ultraviolet light (UVB) treatment during the 4 weeks prior to collecting Screening NRS scores.
11. Has had a history of substance abuse within 6 months prior to completing Screening.
12. Has received opiates on a daily basis during the 2 weeks prior to Study Drug administration.
13. Has known hypersensitivity or allergy to nalbuphine or vehicle components. Has a known drug allergy to opioids.
14. Received any other investigational drug within 4 weeks of Study Drug administration.
15. Recent hospitalization for clinically significant nonelective medical problem within 2 weeks prior to collecting Screening NRS scores.
16. Is a pregnant or lactating female.

### Outcomes

The primary endpoint was the change from Baseline to the Evaluation Period (Study weeks 7 and 8) in the modified intent-to-treat population. Quality of life-related secondary endpoints included change from Day 1 to the Evaluation Period in itching-related quality of life (using the Skindex-10) and itching-related sleep disruption (using the Itch Medical Outcomes Study [Itch MOS], SLP-9). The Hospital Anxiety and Depression Score (HADS) used as a general measure of anxiety and depression. All patient-reported outcomes measures were selected based on their prior validation in uremic pruritus patients specifically or in the dialysis population more generally.

### Skindex-10 Questions

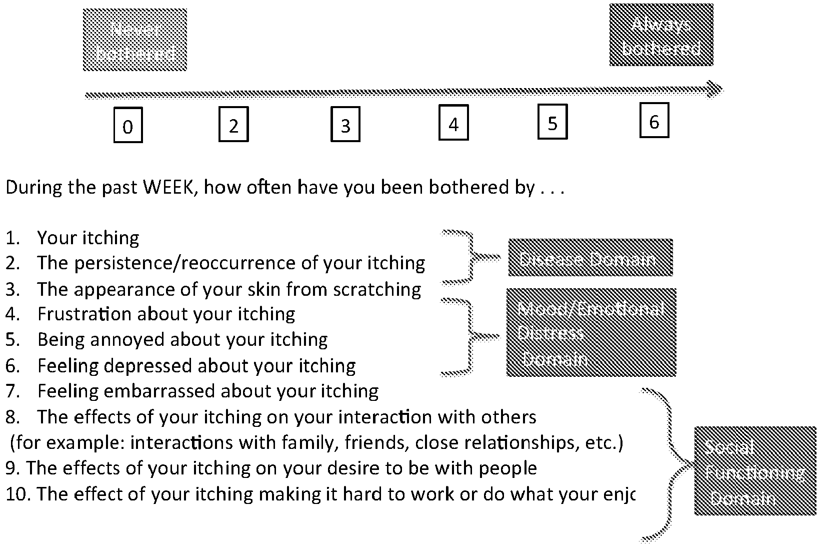

### Itch Medical Outcomes Study (MOS) Questions

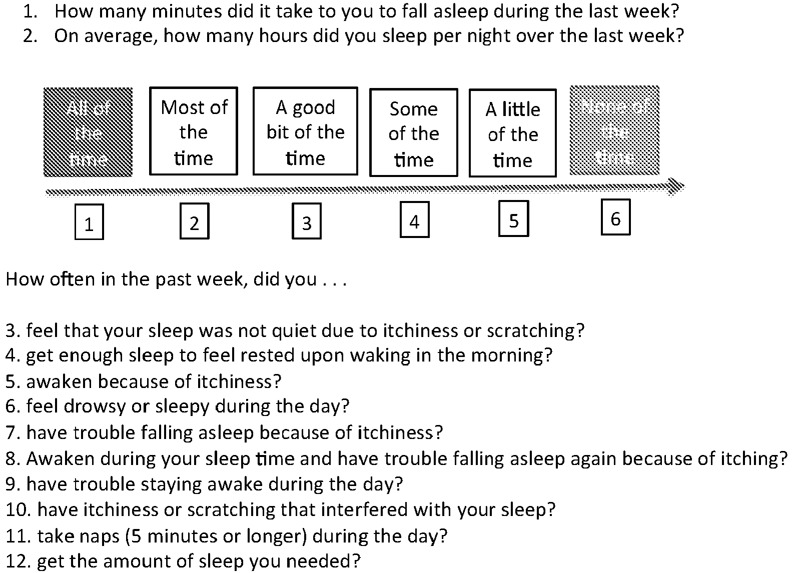

### Statistical Methods

The modified intent-to-treat MITT population consisted of all randomized patients who had a baseline calculated NRS and at least one post-baseline NRS during the 2-week Titration or 6-week Stable Dose Period and was the pre-specified population for all efficacy analyses. Patients were analyzed by group to which they were randomized.

The primary and quality of life secondary endpoint analyses used a mixed model repeated measures ANCOVA with the main effects of treatment and site and baseline worst itch NRS score as the covariate. The model included time (i.e. Visit) as a factor variable and treatment*time (with placebo as the reference category) with an unstructured covariance structure for repeated measures. Data from all post-baseline visits were used to fit the model. For the primary endpoint, the baseline value was defined as the mean of worst itch daytime and nighttime NRS scores used for satisfying the protocol inclusion criterion #5 (mean worst itching NRS ≥ 4.5 based on a total of 3 daytime and 3 nighttime measurements from 3 dialysis visits). For the secondary endpoints, the baseline value was based on a measurement taken on Study Day 1, prior to randomization. A pre-specified step-down procedure was used. The first comparison was of nalbuphine 120 mg BID dose vs. placebo. If the first comparison was significant at the p<0.05 level, then the comparison between the nalbuphine 60 mg BID dose vs. placebo would be conducted. Patients with a 15% or greater response in worst itch NRS were analyzed using a Cochran-Mantel-Haenszel (CMH) test. Analysis of the Itch MOS utilized the SLP-9 scoring algorithm.

### Interventions

In the first 2 weeks of treatment, patients were blindly force-titrated to their assigned target dose, with the patients in the active arms reaching a dose of 60 mg BID (NAL 60) after the first week and, for those in the high dose group, to 120 mg BID (NAL 120) after the second week. Study drug was administered in blister cards containing the labeled morning and evening doses for each day of the week. Subsequently, patients continued stable doses of the study drug for an additional 6 weeks and were then washed off study drug and followed for another 2 weeks for itching intensity and safety. No down-titration was permitted during the study, although patients who missed 6 or more consecutive doses during the Stable Dose Period (Weeks 3 - 8) could, with the Medical Monitor's approval, re-start treatment with blinded re-titration of just the morning or evening dose for 3 days before returning to the original dose. Patients were allowed to remain on their background antipruritic medications such as antihistamines and the use of these medications and indication for treatment was collected.

### Sample Size Calculations

The sample size of 120 per treatment arm (in the modified intent-to-treat population) was based on 90% power and two-sided significance testing at the α = 0.05 level using a two-sample t-test to detect a difference of 1.5 points with a SD of 3.5 or difference of 1.25 with a SD of 3.0.

### Randomization

Randomization was performed by site personnel, using an interactive web-based randomization system (IWRS), which assigned unique blister card numbers reflecting the blinded treatment assignment. The randomization allocation sequence was generated by a contracted research organization.

### Results

Screening for the study was initiated in June 2014 and patients were randomized during the period June 2014 - March 2015. Screening was halted once it was estimated that the number of patients in Screening would yield the remaining number of planned randomized patients (approximately 360). A total of 597 patients were screened, 373 were randomized, and 317 were evaluated for efficacy.

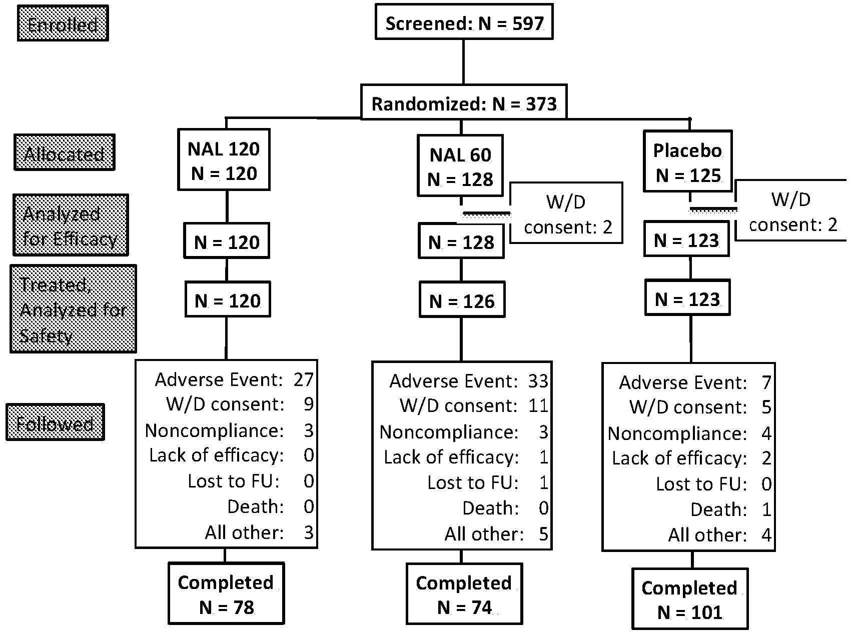

Fifty percent of the population had severe pruritus (NRS ≥7.0) and the mean duration of itching had been 3.2 (2.9) years. Demographics, baseline comorbidities, dialysis adequacy, access type, and vintage, calcium, phosphate, hemoglobin, and parathyroid hormone levels were generally balanced across the three treatment arms (**Table 3**), although the percentage of patients with diabetes and ischemic heart disease interventions was higher in the NAL 60 group compared with the other groups.

**Table 3: Baseline Characteristics**

| | **Nalbuphine 120 mg BID (N = 120)** | **Nalbuphine 60 mg BID (N = 128)** | **Placebo BID (N = 123)** |
|---|---|---|---|
| | | | |
| Age (years) | 55 (12) | 55 (12) | 56 (12) |
| Gender (% male) | 58 | 54 | 60 |
| Race (White/Black/Asian) (%) | 53/47/0 | 45/52/1 | 48/49/1 |
| Hemodialysis Duration (years) | 4.7 (4.2) | 4.8 (4.0) | 4.5 (4.4) |
| Diabetes (%) | 50 | 56 | 48 |
| Congestive Heart Failure (%) | 33 | 25 | 29 |
| Peripheral vascular disease | 14 | 16 | 12 |
| Peripheral vascular disease intervention' (%) | 2 | 6 | 4 |
| Myocardial infarction (%) | 8 | 12 | 17 |
| Ischemic heart disease intervention (%) | 15 | 21 | 7 |
| Hemodialysis Access (AVF/AVG/tunneled catheter) % | 73/18/9 | 75/15/8 | 70/18/11 |
| Urea reduction ratio (%) | 74 (5.5) | 74 (5.5) | 75 (5.7) |
| Kt/V | 1.6 (0.5) | 1.6 (0.3) | 1.6 (0.3) |
| Intact Parathyroid Hormone (iPTH, pg/mL) | 452 (455) | 382 (318) | 464 (390) |
| Phosphate (mg/dL) | 5.6 (1.5) | 5.4 (1.8) | 5.7 (1.8) |
| Calcium (mg/dL) | 8.98 (0.90) | 8.99 (0.79) | 9.13 (0.87) |
| Hemoglobin (g/dL) | 10.6 (0.14) | 10.6 (0.11) | 10.9 (0.11) |
| Patient-Assessed Disease Severity Type C | 37.6% | 38.5% | 37.6% |

| | | | |
|---|---|---|---|
| ¹ Excluding dialysis access-related procedures. Data presented are means (SD) or percentages | | | |

The primary efficacy endpoint was met. From a mean baseline NRS of 6.9 (1.5), the mean NRS declined by (absolute reduction of -3.5 [2.4]) in the NAL 120 group. This difference was statistically different than placebo (p = 0.017) (**Table 4,** **Figure 1**). There was no significant difference between NAL 60 vs. placebo. A significant separation between the NAL 120 group and placebo group was evident starting in the week following the blinded titration, with no apparent development of tolerance during the 8-week treatment period (**Figure 2**). NRS scores increased during the off-drug washout period. There was no increase in the percentage of patients using antipruritic medications over time (**Figure 3**). The majority of antipruritic medications used were antihistamine (21% of all subjects). Additionally, 4.6% received corticosteroids and 0.5% received gabapentin for pruritus.

**Table 4: Primary Efficacy Endpoint - Worst Itching Intensity**

| | **Nalbuphine 120 mg BID (N = 120)** | **Nalbuphine 60 mg BID (N = 128)** | **Placebo BID (N = 123)** |
|---|---|---|---|
| Baseline NRS (mean, SD) | 6.9 (1.5) | 6.9 (1.4) | 6.8 (1.4) |
| Change from Baseline to the Evaluation Period NRS (mean SD) | -3.5 (2.4) | -3.1 (2.4) | -2.8 (2.2) |
| Difference in the Change from Baseline to the Evaluation Period (LSMEAN, SE, 95% CI) | -0.73 (0.31) | -0.24 (0.31) | |
| | (-1.34, -0.13) p = 0.017 | (-0.84, 0.37) p = 0.441 | |
| p-values are vs. placebo. A greater decline in scores reflects greater reduction in itching intensity. | | | |

Secondary endpoints included 2 measures of quality of life related to itching and one general measurement of anxiety and depression. There were no statistically significant differences in either active group vs. placebo on the total Skindex-10, Itch MOS, or HADS instruments (**Table 5**), although a trend for less sleep disruption (p = 0.062) and lesser bothersomeness of itch (Skindex-10 Disease Domain, p = 0.053) were noted in pre-specified analyses comparing the NAL 120 group with placebo. Sleep latency (time to fall asleep), assessed on the Itch MOS, was >30 minutes in 67.5%, 68.7%, and 67.5% of patients, respectively in the NAL 120, NAL 60 mg, and placebo groups at baseline respectively, and 25.0%, 35.9%, and 53.7% of patients, respectively, during the last treatment week (Week 8). A ≥15% change from baseline to the Evaluation Period in the NRS occurred in 84.5%, 80.5%, and 78.7% of patients in the NAL 120, NAL 60, and placebo groups (p=NS).

**Table 5: Quality of Life-Related Secondary Efficacy Endpoints**

| | **Nalbuphine 120 mg BID (N = 120)** | **Nalbuphine 60 mg BID (N = 128)** | **Placebo BID (N = 123)** |
|---|---|---|---|
| | **Change from Baseline to the Evaluation Period** | | |
| Skindex-10 (Itch-Related Quality of Life) | | | |
| Total Score [0 (best) to 60 (worst)] | -17.0 (14.5) | -13.8 (14.6) | -15.0 (14.1) |
| Disease Domain (bothersomeness of itching) | -6.4 (4.4)§ | -5.3 (4.9) | -5.2 (4.3) |
| (0-18) | | | |
| Mood/emotional distress domain (0-24) | -5.2 (5.2) | -4.4 (5.0) | -4.9 (4.9) |
| Social Functioning Domain (0-18) | -5.4 (6.5) | -4.2 (6.7) | -5.0 (6.5) |

| Itch MOS (Itch-Related Sleep Disruption) | | | |
|---|---|---|---|
| ItchMOS [0 (best) to 60 (worst)] | -16.0 (19.7)§ | -13.9 (17.6) | -12.2 (17.7) |

| Hospital Anxiety and Depression Scale (HADS) | | | |
|---|---|---|---|
| Anxiety Subscale (0 - 21) | -1.64 (3.1) | -1.72 (3.0) | 1.89 (3.1) |
| Depression Subscale (0 - 21) | -1.03 (3.5) | -0.68 (3.1) | -1.31 (3.2) |
| Data are mean (SD), p-values are values are vs. placebo. A decline in scores reflects quality of life improvement.. §p<0.1, *p<0.05 | | | |

In a post-hoc analysis of the subgroup with severe uremic pruritus (Baseline NRS ≥7.0, n = 179, **Table 6**), mean itching intensity in the NAL 120 group decreased by 55%, with an absolute reduction of 4.5 (2.5) from a baseline of 8.2 (0.8) (p = 0.007 vs. placebo). Sleep disruption due to itching improved significantly relative to placebo (p = 0.007). Neither itching intensity nor Itch MOS improved significantly in the NAL 60 group compared with placebo.

**Table 6: Subgroup Analysis of Patients with Severe Pruritus (Baseline NRS ≥ 7)**

| | **Nalbuphine 120 mg BID (N = 63)** | **Nalbuphine 60 mg BID (N =61)** | **Placebo BID (N = 55)** |
|---|---|---|---|
| Worst Itching Intensity NRS (Baseline) [0 (no itching) - 10 (worst possible itching) | 8.2 (0.8) | 8.0 (0.9) | 8.0 (0.9) |
| | **Change from Baseline to the Evaluation Period** | | |

| Worst Itching Intensity Numerical Rating Scale (NRS) | | | |
|---|---|---|---|
| NRS [0 (no itching) -10 (worst possible itching) | -4.5 (2.5)** | -3.4 (2.6) | -3.2 (2.7) |

| Skindex-10 (Itch-Related Quality of Life) | | | |
|---|---|---|---|
| Total Score [0 (best) to 60 (worst)] | -20.5 (15.7) | -16.4 (15.1) | -17.2 (16.6) |
| Disease Domain (bothersomeness of itching) [0 - 18] | -7.1 (4.8)* | -6.2 (5.1) | 5.4 (5.0) |
| Mood/Emotional Distress Domain [0 - 24] | -6.4 (5.7)§ | -5.1 (5.0) | -5.2 (5.6) |
| Social Functioning Domain [0-18] | -7.1 (6.9) | -5.1 (7.04) | -6.6 (7.4) |

| Itch MOS (Itch-Related Sleep Disruption) | | | |
|---|---|---|---|
| Itch MOS (Itch-Related Sleep Disruption) [0 (best) to 60 (worst)] | -21.6 (21.5)** | -15.0 (17.7) | -11.4 (22.5) |
| Data are mean (SD), p-value are values are vs. placebo. A decline in scores reflects quality of life improvement. §p<0.1, *p<0.05; **p<0.01 | | | |

In the NAL 120, NAL 60, and placebo groups, 65%, 58%, and 81% completed the 8-week treatment. The most common reason for discontinuing treatment in the active groups were due to opioid type side effects (e.g. nausea and vomiting) that occurred during the forced titration period. There was one death, which occurred in the placebo group. The incidence of serious adverse events was 6.7%, 12.7%, and 15.4% in the NAL 120 mg, NAL 60 mg, and placebo groups respectively (**Table 7**). There were no reports of euphoric or mood-elevating type adverse events or respiratory depression.

**Table 7: Adverse Events**

| | | **Nalbuphine 120 mg BID (N = 120)** | **Nalbuphine 60 mg BID (N = 128)** | **Placebo BID (N = 123)** |
|---|---|---|---|---|
| Deaths (N) | | 0 | 0 | 1 |
| Serious Adverse Events | | 6.7% | 12.7% | 15.4% |
| • | Related serious adverse events (N) | 1 (vertigo) | 0 | 0 |
| Adverse Events Leading to Discontinuation' | | 27 (22.5%) | 33 (26.2%) | 7 (5.7%) |
| Nausea | | 10.0% | 9.5% | 0 |
| Vomiting | | 5.0% | 9.5% | 2.4% |
| Somnolence | | 1.7% | 4.0% | 0 |
| Dizziness | | 2.5% | 0.8% | 0 |
| Hallucination | | 2.5% | 1.6% | 0 |
| ¹Discontinuation events occurring in >2 patients in any group are shown | | | | |

### Discussion

We report the results of the largest randomized controlled trial conducted to date in uremic pruritus. The trial met its primary endpoint, demonstrating a significant and durable reduction in itch intensity in the NAL 120 mg group vs. placebo in hemodialysis patients with moderate and severe uremic pruritus receiving background antipruritic drugs such as antihistamines and corticosteroids. The 49% reduction in itching intensity was accompanied by trends in improvement in the quality of life measurement most proximate to itching intensity (bothersomeness of itching) as well as on sleep disruption due to itching. These findings point to clinical benefit of the observed reduction in itching intensity observed in the NAL 120 group. In prior studies in chronic pruritus, patients differentiated changes in itching intensity of 25-30% with changes in their verbally reported description of itching as "mild", "moderate", and "severe". In uremic pruritus studies, specifically, changes in VAS on a 100 mm scale of 25 mm (the equivalent of a 2.5 reduction on a 0-10 NRS scale) following treatment with nalfurafine, resulted in significantly increased the number of nights with sound sleep and days with nondisturbing itch. Reductions in itching intensity and improvements in sleep disruption were qualitatively similar, but of greater magnitude and statistical significance, among the subgroup of 179 patients with severe pruritus whose mean baseline NRS was approximately 8. These findings are encouraging as patients with severe pruritus have the greatest disease burden; however, because this analysis was not pre-specified, confirmation in future controlled trials is needed. The effects of NAL 120 mg on reducing sleep latency and disruption appear not to be due to a general sedative effect, but, rather, to an effect of itching intensity. Statistically significant correlations between the change from baseline in NRS and all but one individual item on the Itch MOS were observed.

We also demonstrated that the efficacy of NAL 120 was evident through 8 weeks of treatment, with no apparent development of tolerance - this finding supports the utility of nalbuphine ER tablets for chronic use.

The background use of emollients and antipruritic medications was allowed in the trial. Whereas rigorous data supporting the use of antihistamines and other drugs for uremic pruritus is lacking, the allowance of such usual care is a strength of the trial in that it demonstrated the effects of nalbuphine ER tablets when added to such care, as would be expected in a real world setting. Our finding that only 20-25% of patients were receiving antipruritic medications despite their severe pruritus is not unexpected. Narita and colleagues have reported similar percentage of antipruritic medication use in their study of 1173 hemodialysis patients (Narita) and others have noted that patients with the most severe pruritus are often paradoxically on no treatments because "nothing works".

Nalbuphine ER tablets appeared to be neutral in mood effects, both as measured by adverse event reporting, the Skindex-10 mood domain, and the HADS. The lack of antidepressant, anxiolytic, or euphoric effects of nalbuphine ER tablets is consistent with the fact that regarding its mu-receptor activity, it is a µ-opioid antagonist rather than µ-opioid agonist.

Placebo response in trials with subjectively reported symptoms is well known but patient and trial factors that contribute to this response are largely unidentified. In a review of neuropathic pain trials, the placebo effect ranged from 11-35% with typical trials having placebo response rates of approximately 27%. We used the neuropathic pain trial literature as a benchmark because neuropathic pain, like uremic pruritus is chronic, caused by neural hypersensitivity, and assessed with similar patient reported outcomes measures (i.e. pain intensity measured with a numerical rating scale or visual analog scale) and there is a larger literature in this area compared to chronic pruritus. Placebo response in uremic pruritus trials has been variable. In one phase 3, multicenter randomized, placebo-controlled trial of intravenous nalfurafine (TRK-820) conducted in Europe in 339 hemodialysis patients, the placebo response exceeded 60% whereas a similarly-sized Japanese Phase 3 trial evaluating oral nalfurafine in hemodialysis patients, the placebo-response rate was approximately 20%. The placebo response in this trial, 40%, was, therefore, within range of prior uremic pruritus trials, but higher than typically observed in neuropathic pain trials.

The safety profile of nalbuphine ER tablets from this trial suggested no adverse safety trends other than those expected from a centrally-acting opioid. The findings that the serious adverse event rate was not higher in either active arm compared with placebo is underscored by the fact that the study was conducted in a complex and ill population of hemodialysis patients. As the study protocol had few medical exclusion criteria , the safety findings from this trial are likely generalizable to the intended target population. There was a relatively high dropout rate related to non-serious nausea and vomiting and, to a lesser extent, to somnolence, dizziness, and hallucinations. The dropout events predominantly occurred when the dose was still being titrated (i.e. dose was less than 60 mg BID). Therefore, these events appeared not to be dose-dependent, but, rather, time-dependent. A lower initiation dose, slower titration, better setting of patient expectations, and greater attention to management of expected gastrointestinal adverse effects may help to reduce dropout rates in future trials.

The primary limitation of the study was the relatively high dropout rate. Because these early dropouts occurred to a disproportionately greater extent in the active arms of the trial, the early time point of discontinuation (i.e., prior to reaching the target dose), and the use of a mixed model to handle missing data, the direction of the bias is expected to be towards a lesser demonstration of the efficacy of the active treatments.

In this multinational, randomized controlled trial, nalbuphine ER tablets administered at a dose of 120 mg twice daily was safe and effective at reducing itching intensity in hemodialysis patients with moderate and severe uremic pruritus. The study suggests nalbuphine, which is classified as a mu-antagonist-kappa-agonist opioid drug is effective in this distressing condition.

### Example 3:

This clinical study was a Phase 2/3 Open Label Extension Study of the randomized, double-blind, 3-parallel arm, placebo controlled dose ranging study described in Example 2. All those subjects who were randomized, and then completed Example 2 study, were eligible to participate in the Extension Study.

The primary endpoint was a description of the overall incidence and nature of Treatment-Emergent AEs (TEAES) with the secondary endpoint a description of the incidence and nature of TEAEs during Treatment Weeks 4 - 24. Exploratory efficacy endpoints related to collecting data on the PRO instruments (NRS, Skindex-10, Itch MOS Sleep, HADS, PADS), the same instruments evaluated in Example 2.

### Number of Patients (Planned and Analyzed):

Of the 373 patients that were randomized to the study of Example 2, 184 subjects both completed the study of Example 2 and enrolled into Extension Study. 167 subjects who enrolled into Extension Study entered the Treatment Period of the study and were exposed to nalbuphine HCl ER tablet administration and are the basis of the study safety population analysis. 17 subjects who were enrolled in the Extension were never dosed, they were categorized as screen failures and they are not part of the analysis.

### Dosing

Patients in Extension Study were titrated over a dose range of 30 mg QD to 120 mg BID based on tolerability and efficacy. The selected dose range was based on Example 2 study whereby patients were titrated from a 30 mg QD dose either to 60 mg BID or a 120 mg BID dose. The highest dose proposed was 120 mg BID (240 mg daily dose), and was well below the highest recommended daily treatment of 160 mg IV (equivalent to 960 mg oral) for the current marketed product.

### Study design:

The study duration for each patient was up to 26 weeks, with up to 24 weeks on study drug. The Extension Study consisted of a Treatment Period (that was followed by a Washout Safety Follow-up Period) and an Observation Period. Patients either entered directly into a drug Treatment Period (NRS >2) or a no-drug Observation Period (NRS ≤ 2) based on their reported NRS scores on the first Visit (Visit 1a). For up to 12 Extended Screening weeks, patients in the no-drug Observation Period could have also transitioned into the drug Treatment Period if their NRS increased to NRS >2. All patients entering the Treatment Period, whether immediately upon study entry or following a period of time in the Observation Period, were titrated to a dose ranging between 30 mg up to 120 mg BID, the highest dose tested in Example 2. Patients who completed the Observation Period and whose NRS did not exceed NRS >2 over the 12 weeks were considered screen failures.

The total study duration for any individual patient was up to 26 weeks. For patients who enter directly into the Treatment Period, the total amount of time on drug did not exceed 24 weeks. For patients who entered the Treatment Period from the Observation Period, the total amount of time spent in the combined two periods of the study could not exceed 24 weeks. All patients on drug treatment had a 2-week washout and safety follow-up period at the end of the dosing period.

The total amount of time in the Observation Period did not exceed 12 weeks. After these 12 Extended Screening weeks, subjects not eligible for the Treatment Period were screen failed from the study.

The three Extension Study periods are summarized in Table 8.

**Table 8: Description of Study Periods**

| **Study Period** | **Study Weeks** | **Duration** |
|---|---|---|
| Observation Period | Patients who did not meet the criteria to start Treatment (NRS =< 2) were followed in Observation Period visits up to 12 weeks. | Up to 12 Extended Screening weeks |
| | • If NRS remained at or below 2 over the 12 weeks, participation in the study ended (OV12), and the patient was screen failed. | |
| | • If NRS exceeded 2 during the 12-week observation period, the patient became eligible to enter the Treatment Period (defined below) | |
| Treatment Period | • For patients directly entering the Treatment Period (NRS >2) as of Visit 1a, treatment Period began with Study Week 1 (Visit 1a) and ended with Study Week 24 | Up to 24 weeks |
| | • For patients entering the Treatment Period after being followed in the Observation Period, the number of weeks on treatment was equal to 24 weeks minus weeks in Observation period. As a result, the end of the Treatment Period varied. | |
| | • The End of Treatment Visit took place after the patient completed the last week of study drug | |
| Washout and Safety Follow-Up Period | The Washout and Safety Follow-up Period was two (2) weeks in duration. | 2 weeks |
| | For patients directly entering the Treatment Period as of Visit 1a, and completing 24 weeks of study drug treatment, the Washout and Safety Follow-up Period took place during weeks 25 and 26. | |
| | For patients entering the Treatment Period after being followed in the Observation Period, the Washout and Safety Follow-up Period took place during the two (2) weeks after they completed the last week of study drug. | |
| | For all patients, the final Visit was scheduled within a week from completion of the Washout and Safety Follow-up Period. | |

An overall study schematic is shown in Figure 4.

### Results:

Figure 5 displays mean worst itch NRS in the safety population by week in study as well as the three subgroups of the safety population divided by baseline itch values of NRS values from zero to less than 4 (0 - <4), NRS values from greater than or equal to 4 but less than 7 (≥4 - < 7) and NRS values greater than or equal to 7 (≥ 7). Mean worst itch was decreased from baseline to all measured time points in the safety population. The decrease in mean worst itch was most pronounced in the subgroup of patients with baseline NRS values of ≥ 7.

Figure 6 displays Skindex-10 in the safety population by week in study as well as the three subgroups of the safety population divided by baseline itch values of NRS values from zero to less than 4 (0 - <4), NRS values from greater than or equal to 4 but less than 7 (≥4 - < 7) and NRS values greater than or equal to 7 (≥ 7). Mean Skindex-10 decreased from baseline to all measured time points in the safety population. The decrease in mean Skindex-10 was most pronounced in the subgroup of patients with baseline NRS values of ≥ 7.

### Discussion

For the primary efficacy objective of evaluating the safety and tolerability of nalbuphine HCl ER tablets for a treatment period that extended up to 24 weeks, the safety findings were consistent with the known safety profile of nalbuphine as summarized in the parenteral nalbuphine package insert as well as nalbuphine given via the oral route of shorter duration based on previous clinical studies conducted by the Sponsor (e.g., Example 2).

Mean worst itch intensity decreased from baseline in all subsequent measured time points in the safety population.

Efficacy analysis undertaken through multiple PRO instruments over time, along with the worst-itch NRS findings, was consistent with a durability of drug effect. That is to say, that the efficacy data is consistent with the conclusion that patients do not develop a tolerance to the anti-pruritic effects of nalbuphine (see, for example, the graphical representations of Worst Itch and Skindex Scores shown in Figures 5 and 6, respectively).

The embodiments described herein and illustrated by the foregoing examples should be understood to be illustrative of the present invention, and should not be construed as limiting.

## Claims

1. Nalbuphine or a pharmaceutically acceptable salt or ester thereof for use in a method of treating uremic pruritus, comprising administering for at least a week to a patient in need thereof, a daily dose of 240 mg of the nalbuphine or pharmaceutically acceptable salt or ester thereof; and titrating the dose of the nalbuphine or pharmaceutically acceptable salt or ester thereof for at least 2 weeks, prior to said administering.

2. The nalbuphine or pharmaceutically acceptable salt or ester thereof for use according to claim 1, wherein after said treating the patient experiences a substantial reduction in itch compared to prior to said treating.

3. The nalbuphine or pharmaceutically acceptable salt or ester thereof for use according to claim 1 or 2, wherein 120 mg of nalbuphine or a pharmaceutically acceptable salt or ester thereof is administered twice a day, or 240 mg of nalbuphine or a pharmaceutically acceptable salt or ester thereof is administered once a day.

4. The nalbuphine or pharmaceutically acceptable salt or ester thereof for use according to any one of claims 1-3, wherein said administering is for about 8 weeks, 10 weeks, 12 weeks, 24 weeks or 50 weeks.

5. The nalbuphine or pharmaceutically acceptable salt or ester thereof for use according to any one of claims 1-4, wherein the patient has moderate or severe uremic pruritus, chronic kidney disease, and/or is a hemodialysis patient.

6. The nalbuphine or pharmaceutically acceptable salt or ester thereof for use according to any one of claims 1-5, wherein titrating comprises administering ascending doses of the nalbuphine or pharmaceutically acceptable salt or ester thereof until a steady state is achieved in the patient or wherein said titrating comprises administering ascending doses of the nalbuphine or pharmaceutically acceptable salt or ester thereof until an effective amount of 120 mg is achieved in the patient

7. The nalbuphine or pharmaceutically acceptable salt or ester thereof for use according to claim 6, wherein said titrating further comprises administering an initial dose of about 15 mg or about 30 mg once or twice a day.

8. The nalbuphine or pharmaceutically acceptable salt or ester thereof for use according to any one of claims 6-7, wherein said titrating comprises administering the anti-pruritus agent in increments ranging from about 15 mg to about 30 mg.

9. The nalbuphine or pharmaceutically acceptable salt or ester thereof for use according to claim 7, wherein said administering twice a day is with an AM dosage and a PM dosage, wherein the PM dosage is higher than or the same as the AM dosage.

10. The nalbuphine or pharmaceutically acceptable salt or ester thereof for use according to any one of claims 6-9, wherein the rate of adverse events after said titration is substantially the same as the rate of adverse events after administering a placebo for the same period of time.

11. The nalbuphine or pharmaceutically acceptable salt or ester thereof for use according to any one of claims 1-10, wherein:
(i) a patient with moderate or severe baseline itch prior to said treating experiences mild itch after said treating; or
(ii) the rate of infection of the patient after said treating is lower than that of the patients prior to said treating.

12. The nalbuphine or pharmaceutically acceptable salt or ester thereof for use according to any one of claims 1-11, wherein after said treating the patient experiences a reduction of itch that is **characterized by**:
(i) at least about a 30% decline, at least about a 40% decline, or at least about a 50% decline in worst itching intensity Numerical Rating Scale (NRS) value; or
(ii) at least about a 10% improvement, at least about a 20% improvement, or at least about a 30% improvement in Skindex-10 total or subscale domain score; or
(iii) at least about a 20% improvement, at least about a 30% improvement, or at least about a 40% improvement in Itch Medical Outcomes Study (MOS) sleep scale.

13. The nalbuphine or pharmaceutically acceptable salt or ester thereof for use according to any one of claims 1-12, further comprising administering at least one additional antipruritic drug; optionally wherein the at least one additional antipruritic drug is selected from the group consisting of antihistamines and corticosteroids.

14. The nalbuphine or pharmaceutically acceptable salt or ester thereof for use according to any one of claims 1-13, wherein the nalbuphine or pharmaceutically acceptable salt or ester thereof is in the form of an extended release oral dosage form.

15. The nalbuphine or pharmaceutically acceptable salt or ester thereof for use according to any one of claims 1-14, wherein the anti-pruritus agent is administered in a formulation comprising nalbuphine hydrochloride, mannitol, hydroxypropyl cellulose, locust bean gum, xanthan gum, calcium sulfate dihydrate, and magnesium stearate.

16. The nalbuphine or pharmaceutically acceptable salt or ester thereof for use according to any one of claims 6-15, wherein the daily dose of nalbuphine or a pharmaceutically acceptable salt or ester thereof is substantially the same during the administering; optionally
wherein said administering is for about 12 weeks, 24 weeks or 50 weeks; and/or wherein the daily dose of nalbuphine or a pharmaceutically acceptable salt or ester is 240 mg during the administering.

17. The nalbuphine or pharmaceutically acceptable salt or ester thereof for use according to any one of claims 1-16, wherein the administering provides a steady state blood plasma concentration of between about 20 and 80 ng/mL, or between about 30 and 70 ng/mL.

18. The nalbuphine or pharmaceutically acceptable salt or ester thereof for use according to claim 17, wherein the daily dose of nalbuphine or a pharmaceutically acceptable salt or ester is 240 mg.

## Patentansprüche

1. Nalbuphin oder pharmazeutisch annehmbares Salz oder Ester davon zur Verwendung in einem Verfahren zur Behandlung von urämischem Pruritus, umfassend das Verabreichen einer Tagesdosis von 240 mg Nalbuphin oder pharmazeutisch annehmbarem Salz oder Ester davon an einen Patienten, der dieser bedarf, für mindestens eine Woche; und Titrieren der Dosis des Nalbuphins oder des pharmazeutisch annehmbaren Salzes oder Esters davon für mindestens 2 Wochen vor dem Verabreichen.

2. Nalbuphin oder pharmazeutisch annehmbares Salz oder Ester davon zur Verwendung nach Anspruch 1, wobei nach dem Behandeln der Patient einen wesentlichen Rückgang des Juckreizes im Vergleich zu vor der Behandlung erlebt.

3. Nalbuphin oder pharmazeutisch annehmbares Salz oder Ester davon zur Verwendung nach Anspruch 1 oder 2, wobei 120 mg Nalbuphin oder pharmazeutisch annehmbares Salz oder Ester davon zweimal täglich verabreicht werden oder 240 mg Nalbuphin oder pharmazeutisch annehmbares Salz oder Ester davon einmal täglich verabreicht werden.

4. Nalbuphin oder pharmazeutisch annehmbares Salz oder Ester davon zur Verwendung nach einem der Ansprüche 1-3, wobei das Verabreichen für etwa 8 Wochen, 10 Wochen, 12 Wochen, 24 Wochen oder 50 Wochen erfolgt.

5. Nalbuphin oder pharmazeutisch annehmbares Salz oder Ester davon zur Verwendung nach einem der Ansprüche 1-4, wobei der Patient mäßigen oder schweren urämischen Pruritus hat, chronische Nierenerkrankung hat und/oder ein Hämodialysepatient ist.

6. Nalbuphin oder pharmazeutisch annehmbares Salz oder Ester davon zur Verwendung nach einem der Ansprüche 1-5, wobei das Titrieren das Verabreichen steigender Dosen an Nalbuphin oder pharmazeutisch annehmbarem Salz oder Ester davon umfasst, bis ein Steady-State bei dem Patienten erzielt wird, oder wobei das Titrieren das Verabreichen steigender Dosen an Nalbuphin oder pharmazeutisch annehmbarem Salz oder Ester davon umfasst, bis eine wirksame Menge von 120 mg bei dem Patienten erreicht ist.

7. Nalbuphin oder pharmazeutisch annehmbares Salz oder Ester davon zur Verwendung nach Anspruch 6, wobei das Titrieren ferner das Verabreichen einer Anfangsdosis von etwa 15 mg oder etwa 30 mg einmal oder zweimal täglich umfasst.

8. Nalbuphin oder pharmazeutisch annehmbares Salz oder Ester davon zur Verwendung nach einem der Ansprüche 6-7, wobei das Titrieren das Verabreichen des Antipruritusmittels in Schritten im Bereich von etwa 15 mg bis etwa 30 mg umfasst.

9. Nalbuphin oder pharmazeutisch annehmbares Salz oder Ester davon zur Verwendung nach Anspruch 7, wobei das Verabreichen zweimal täglich mit einer Morgendosis und einer Abenddosis erfolgt, wobei die Abenddosis höher ist als oder genauso hoch wie die Morgendosis ist.

10. Nalbuphin oder pharmazeutisch annehmbares Salz oder Ester davon zur Verwendung nach einem der Ansprüche 6-9, wobei Nebenwirkungsrate nach der Titration im Wesentlichen die gleiche ist wie die Nebenwirkungsrate nach dem Verabreichen eines Placebos für den gleichen Zeitraum.

11. Nalbuphin oder pharmazeutisch annehmbares Salz oder Ester davon zur Verwendung nach einem der Ansprüche 1-10, wobei:
(i) ein Patient mit mäßigem oder schwerem Ausgangsjuckreiz vor der Behandlung nach der Behandlung einen leichten Juckreiz erlebt; oder
(ii) die Infektionsrate des Patienten nach der Behanldung niedriger ist als jene des Patienten vor der Behandlung.

12. Nalbuphin oder pharmazeutisch annehmbares Salz oder Ester davon zur Verwendung nach einem der Ansprüche 1-11, wobei nach der Behandlung der Patient einen Rückgang des Juckreizes erlebt, der **gekennzeichnet ist durch**:
(i) eine Abnahme von mindestens etwa 30 %, eine Abnahme von mindestens etwa 40 % oder eine Abnahme von mindestens etwa 50 % beim NRS-Wert der schlimmsten Juckintensität (NRS - Numeric Rating Scale); oder
(ii) eine Besserung von mindestens etwa 10 %, eine Besserung von mindestens etwa 20 % oder eine Besserung von mindestens etwa 30 % bei der Skindex-10-Gesamt- oder Subskala-Domänenpunktzahl; oder
(iii) eine Besserung von mindestens etwa 20 %, eine Besserung von mindestens etwa 30 % oder eine Besserung von mindestens etwa 40 % in der Itch-MOS-Schlafskala (MOS - Medical Outcomes Study).

13. Nalbuphin oder pharmazeutisch annehmbares Salz oder Ester davon zur Verwendung nach einem der Ansprüche 1-12, ferner umfassend das Verabreichen mindestens eines zusätzlichen juckreizstillenden Arzneimittels; wobei das mindestens eine zusätzliche juckreizstillende Arzneimittel gegebenenfalls ausgewählt ist aus der Gruppe bestehend aus Antihistaminika und Kortikosteroiden.

14. Nalbuphin oder pharmazeutisch annehmbares Salz oder Ester davon zur Verwendung nach einem der Ansprüche 1-13, wobei das Nalbuphin oder pharmazeutisch annehmbare Salz oder Ester davon in Form einer oralen Darreichungsform mit verlängerter Freisetzung vorliegt.

15. Nalbuphin oder pharmazeutisch annehmbares Salz oder Ester davon zur Verwendung nach einem der Ansprüche 1-14, wobei das Antipruritusmittel in einer Formulierung verabreicht wird, die Nalbuphinhydrochlorid, Mannit, Hydroxypropylcellulose, Johannisbrotkernmehl, Xanthan, Calciumsulfatdihydrat und Magnesiumstearat umfasst.

16. Nalbuphin oder pharmazeutisch annehmbares Salz oder Ester davon zur Verwendung nach einem der Ansprüche 6-15, wobei die Tagesdosis an Nalbuphin oder einem pharmazeutisch annehmbaren Salz oder Ester davon während der Verabreichung im Wesentlichen die gleiche ist; wobei das Verabreichen gegebenenfalls etwa 12 Wochen, 24 Wochen oder 50 Wochen lang erfolgt; und/oder wobei die Tagesdosis an Nalbuphin oder einem pharmazeutisch annehmbaren Salz oder Ester während der Verabreichung bei 240 mg liegt.

17. Nalbuphin oder pharmazeutisch annehmbares Salz oder Ester davon zur Verwendung nach einem der Ansprüche 1-16, wobei das Verabreichen eine Steady-State-Blutplasmakonzentration von zwischen etwa 20 und 80 ng/ml oder zwischen etwa 30 und 70 ng/ml bereitstellt.

18. Nalbuphin oder pharmazeutisch annehmbares Salz oder Ester davon zur Verwendung nach Anspruch 17, wobei die Tagesdosis an Nalbuphin oder einem pharmazeutisch annehmbaren Salz oder Ester bei 240 mg liegt.

## Revendications

1. Nalbuphine ou sel ou ester pharmaceutiquement acceptable correspondant pour une utilisation dans un procédé de traitement du prurit urémique, comprenant une administration pendant au moins une semaine, à un patient qui en a besoin, d'une dose quotidienne de 240 mg de la nalbuphine ou du sel ou de l'ester pharmaceutiquement acceptable correspondant ; et le titrage de la dose de la nalbuphine ou du sel ou de l'ester pharmaceutiquement acceptable correspondant pendant au moins 2 semaines, avant ladite administration.

2. Nalbuphine ou sel ou ester pharmaceutiquement acceptable correspondant pour une utilisation selon la revendication 1, après ledit traitement, le patient éprouvant une réduction substantielle de démangeaisons par comparaison avec l'avant ledit traitement.

3. Nalbuphine ou sel ou ester pharmaceutiquement acceptable correspondant pour une utilisation selon la revendication 1 ou 2, 120 mg de nalbuphine ou d'un sel ou d'un ester pharmaceutiquement acceptable correspondant étant administrés deux fois par jour, ou 240 mg de nalbuphine ou d'un sel ou d'un ester pharmaceutiquement acceptable correspondant étant administrés une fois par jour.

4. Nalbuphine ou sel ou ester pharmaceutiquement acceptable correspondant pour une utilisation selon l'une quelconque des revendications 1 à 3, ladite administration étant pendant environ 8 semaines, 10 semaines, 12 semaines, 24 semaines ou 50 semaines.

5. Nalbuphine ou sel ou ester pharmaceutiquement acceptable correspondant pour une utilisation selon l'une quelconque des revendications 1 à 4, le patient ayant un prurit urémique modéré ou grave, une maladie rénale chronique et/ou étant un patient sous hémodialyse.

6. Nalbuphine ou sel ou ester pharmaceutiquement acceptable correspondant pour une utilisation selon l'une quelconque des revendication 1 à 5, le titrage comprenant une administration de doses croissantes de la nalbuphine ou du sel ou de l'ester pharmaceutiquement acceptable correspondant jusqu'à ce qu'un état stable soit atteint chez le patient ou ledit titrage comprenant une administration de dose croissante de la nalbuphine ou du sel ou de l'ester pharmaceutiquement acceptable correspondant jusqu'à ce qu'une quantité efficace de 120 mg soit atteinte chez le patient.

7. Nalbuphine ou sel ou ester pharmaceutiquement acceptable correspondant pour une utilisation selon la revendication 6, ledit titrage comprenant en outre une administration d'une dose initiale d'environ 15 mg ou d'environ 30 mg une ou deux fois par jour.

8. Nalbuphine ou sel ou ester pharmaceutiquement acceptable correspondant pour une utilisation selon l'une quelconque des revendication 6 et 7, ledit titrage comprenant une administration de l'agent anti-prurit en incréments dans la plage d'environ 15 mg à environ 30 mg.

9. Nalbuphine ou sel ou ester pharmaceutiquement acceptable correspondant pour une utilisation selon la revendication 7, ladite administration deux fois par jour étant avec un dosage le matin et un dosage l'après-midi, le dosage de l'après-midi étant supérieur ou identique au dosage du matin.

10. Nalbuphine ou sel ou ester pharmaceutiquement acceptable correspondant pour une utilisation selon l'une quelconque des revendications 6 à 9, le taux d'effets indésirables après ledit titrage étant sensiblement le même que le taux d'effets indésirables après une administration d'un placébo pendant la même période de temps.

11. Nalbuphine ou sel ou ester pharmaceutiquement acceptable correspondant pour une utilisation selon l'une quelconque des revendications 1 à 10,
(i) un patient ayant des démangeaisons de ligne de base modérées ou graves avant ledit traitement éprouvant de légères démangeaisons après ledit traitement ; ou
(ii) le taux d'infection du patient après ledit traitement étant inférieur à celui des patients avant ledit traitement.

12. Nalbuphine ou sel ou ester pharmaceutiquement acceptable correspondant pour une utilisation selon l'une quelconque des revendications 1 à 11, après ledit traitement, le patient éprouvant une réduction de démangeaisons qui est **caractérisée par** :
(i) un déclin d'au moins environ 30 %, un déclin d'au moins environ 40 % ou un déclin d'au moins environ 50 % de la valeur de l'échelle d'évaluation numérique (NRS) de la pire intensité de démangeaisons ; ou
(ii) une amélioration d'au moins environ 10 %, une amélioration d'au moins environ 20 % ou une amélioration d'au moins environ 30 % du score du domaine total ou de sous-échelle Skindex-10 ; ou
(iii) une amélioration d'au moins environ 20 %, une amélioration d'au moins environ 30 % ou une amélioration d'au moins environ 40 % de l'échelle de sommeil de l'étude de résultats médicaux (MOS) de démangeaisons.

13. Nalbuphine ou sel ou ester pharmaceutiquement acceptable correspondant pour une utilisation selon l'une quelconque des revendications 1 à 12, comprenant en outre une administration d'au moins un médicament antipruritique supplémentaire ; éventuellement, l'au moins un médicament antipruritique supplémentaire étant choisi dans le groupe constitué par des antihistaminiques et des corticostéroïdes.

14. Nalbuphine ou sel ou ester pharmaceutiquement acceptable correspondant pour une utilisation selon l'une quelconque des revendications 1 à 13, la nalbuphine ou le sel ou l'ester pharmaceutiquement acceptable correspondant étant sous la forme d'une forme de dosage orale à libération étendue.

15. Nalbuphine ou sel ou ester pharmaceutiquement acceptable correspondant pour une utilisation selon l'une quelconque des revendications 1 à 14, l'agent anti-prurit étant administré dans une formulation comprenant du chlorhydrate de nalbuphine, du mannitol, une hydroxypropylcellulose, une gomme de caroube, une gomme xanthane, du dihydrate de sulfate de calcium et du stéarate de magnésium.

16. Nalbuphine ou sel ou ester pharmaceutiquement acceptable correspondant pour une utilisation selon l'une quelconque des revendications 6 à 15, la dose quotidienne de nalbuphine ou d'un sel ou d'un ester pharmaceutiquement acceptable correspondant étant sensiblement la même pendant l'administration ; éventuellement, ladite administration étant pendant environ 12 semaines, 24 semaines ou 50 semaines ; et/ou la dose quotidienne de nalbuphine ou d'un sel ou d'un ester pharmaceutiquement acceptable étant de 240 mg pendant l'administration.

17. Nalbuphine ou sel ou ester pharmaceutiquement acceptable correspondant pour une utilisation selon l'une quelconque des revendications 1 à 16, l'administration fournissant une concentration de plasma sanguin à l'état d'équilibre comprise entre environ 20 et 80 ng/mL ou entre environ 30 et 70 ng/mL.

18. Nalbuphine ou sel ou ester pharmaceutiquement acceptable correspondant pour une utilisation selon la revendication 17, la dose quotidienne de nalbuphine ou d'un sel ou d'un ester pharmaceutiquement acceptable correspondant étant de 240 mg.
